# EUROPEAN PATENT SPECIFICATION

(11) **EP 0 398 472 B1**
(45) Date of publication and mention of the grant of the patent: **30.11.1994**
(21) Application number: 90302867.8
(22) Date of filing: 16.03.1990
(51) Int. Cl.: C08G 18/38, A61K 9/32

(54) **Biodegradable polyurethanes**
Biologisch abbaubare Polyurethane
Polyuréthanes biodegradables

(30) Priority: 17.03.1989 JP 65136/89
(43) Date of publication of application: 22.11.1990
(73) Proprietor: ONO PHARMACEUTICAL CO., LTD., Chuo-ku Osaka 541 (JP)
(72) Inventor: Kimura, Yoshiharu, Ohmihachiman-shi, Siga (JP); Kim, Soonih, c/o Minase Research Institute, Mishima-gun, Osaka (JP); Nishiura, Akio, c/o Minase Research Institute, Mishima-gun, Osaka (JP)
(74) Representative: Bentham, Stephen

(56) References cited:
- EP-A- 0 016 654
- EP-A- 0 109 624
- US-A- 3 909 497
- US-A- 4 235 988
- US-A- 4 663 308

## Description

The present invention relates to novel polyurethanes which are degradable in the large intestine, processes for their preparation and pharmaceutical adjuvants containing them. More particularly, the invention relates to polyurethanes which are degraded by azo-reducing enzymes produced by a large number of intestinal bacteria, existing in the large intestine, processes for their preparation and pharmaceutical adjuvants for oral administration, containing the said polyurethanes.

In general, it has been found that a medicament administered orally is absorbed in the small intestine and only residual amounts reach the lower part of the small intestine and the large intestine (colon and rectum). One approach used to deliver a medicament to the lower part of the small intestine and the large intestine is a pharmaceutical composition with a pH-sensitive coating. In such a composition, the coating dissolves in response to a rise in pH and the medicament in the composition is then released. However, pH of digestive organs in the body changes during the day, varies according to food intake and also varies naturally between individuals. Accordingly, the medicament of the composition may be unexpectedly released and thus absorbed in the upper part of the small intestine or the composition is not dissolved at all and is excreted without release of the medicament.

Another idea for transmitting a medicament to the large intestine which has been proposed is a chemically modified prodrug. However, it has the drawback that it is also absorbed in the upper part of the small intestine. Therefore, such a medicament must be administered in large amount in order that it is transmitted to the large intestine at a high enough concentration and consequently its side effects are increased. Furthermore, with a prodrug which is metabolized to produce an active drug, the released protecting/modifying group may cause side effects. It has been found that the modifying group of salicylazosulfapyridine (a prodrug of 5-aminosalicylic acid (Peppercorn M.A. et al, J-Pharmacol. Exp. Ther. 181: 555-562, 1972)) sulfapyridine, has toxicity (International Pharmacy Journal, 1(16), 223-226, 1987).

Furthermore, it has been found that peptides such as insulin, vasopressin etc, administered orally are degraded and inactivated by digestive enzymes such as peptidase in the small intestine, and their bioavailability is thus extremely low. It has been suggested that absorption from the large intestine of such peptides in which digestive enzymes hardly exist, may be effective. However, no pharmaceutical composition for oral administration for such a purpose has been developed.

Metabolism in the large intestine is almost exclusively conducted by intestinal bacteria, which is exclusively conducted by intestinal bacteria, which is different from other digestive organs. In order to obtain high molecular weight compounds for coating which are degraded specifically in the large intestine, their structure should be effectively constituted by considering the action of the intestinal bacteria. For example, cellulose, which is known to be metabolized by Eschericia coli, has been an essential factor for molecular weight compounds degraded in the large intestine, and has been utilized in various ways. However, cellulose and its derivatives are difficult to process and it is difficult to control parameters such as their rate of degradation.

One chemical bond severed by the action of intestinal bacteria is an aromatic azo bond. It is known that the bond is reduced to an amino group by an azo-reducing enzyme released by intestinal bacteria as follows: 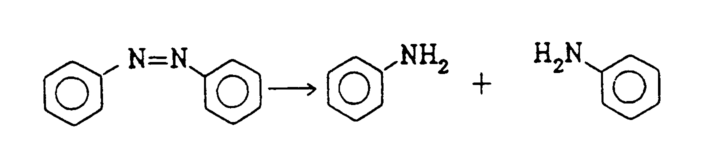
A coating polymer for delivering a medicament to the lower part of the digestive organ, by using such an enzymatic reaction, is proposed in United States Patent No. 4,663,308. It is polymer from ethylenically unsaturated monomers (e.g. vinyl acetate, acrylic acid, methacrylic acid etc.) as main chain, cross-linked by a divinylazobenezene of the formula: 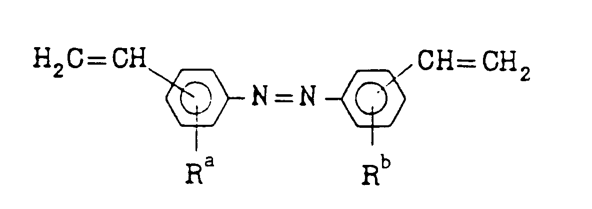
wherein R^{a} and R^{b} are each, independently, hydrogen, halogen, nitro, or other substituents.

However, the cross-linked polymer is unstable to light and it is difficult to achieve high conversion of monomers to polymer. Therefore, it has such defects that its solubility in various solvents is low and that its film forming properties are poor. Furthermore, it is difficult to positively design the molecular structure to control the rate of release of an associated drug or the like.

As a result of energetic investigations, we have now found that polyurethanes (I) of the composition defined below which have azo group(s) in their main chain, are high molecular weight compounds for coating which have an excellent film forming property and degrade specifically in the large intestine.

The polyurethanes (I) of the present invention have azo group(s) in main chain, and are not vinyl polymers but polyurethanes. The polymers of the present invention are therefore very different in structure from the polymers disclosed in U.S.A-4,663,308 and are novel. Furthermore, the present polymers have excellent pharmaceutical properties, not suggested by the polymers in the above U.S. Patent.

Accordingly, the present invention provides:
polyurethanes (I) of average molecular weight of 1000 to 100000, comprising plural segments, which are each structural units of A-B, A-C and A-D of the formulae: 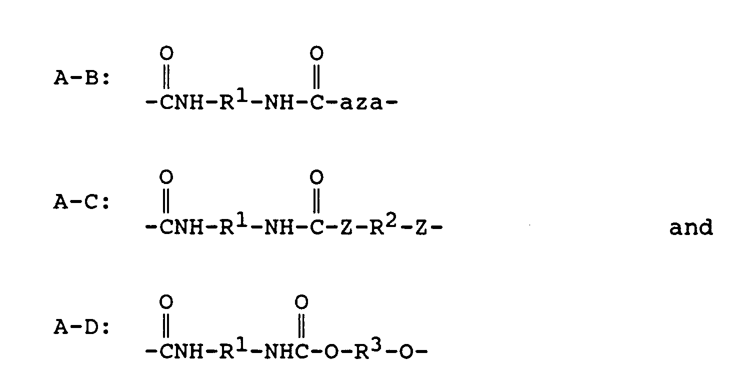
the molar proportion of the said segments of A-B, A-C and A-D i.e. x : y : z being 0.01 to 0.8 : O to 0.80 : 0 to 0.99, provided that the sum of x, y and z is 1.0;
the segments A, B, C and D being structural units of the formulae:

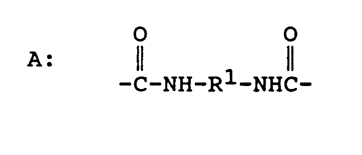

B: -aza-

C: -Z-R²-Z- and

D: -O-R³-O-;

wherein R¹ is a skeleton of a diisocyanate and the three R¹ groups in each of the segments A-B, A-C and A-D are same or different;
aza is a group of the formula: 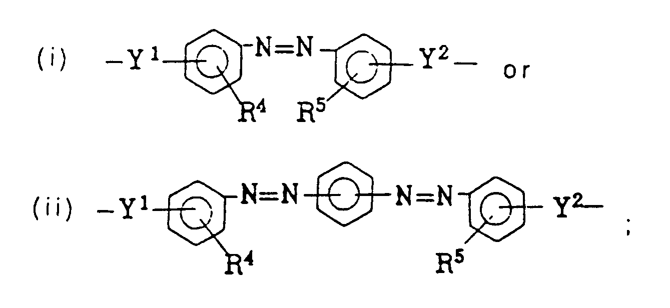
wherein Y¹ and Y², which may be the same or different, represent oxygen, imino (-NH-) or a group of the formula:
*R⁶-O, *R⁶-NH, O-R⁶-O, *NHCO-R⁶-NH or *CONH-R⁶-NH;
wherein R⁶ is alkylene and the atom or the end of the group marked by * is bonded to the phenyl ring;
R⁴ and R⁵ are each, independently, hydrogen, halogen, nitro or phenyl;
R² is a polyalkylene glycol residue;
Z is oxygen or imino and both groups Z are the same;
R³ is alkylene or a group of the formula:
R⁷-O-R⁸, R⁷-NH-R⁸ or 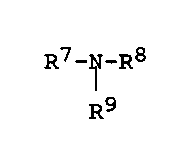
wherein R⁷ and R⁸, which may be the same or different, are alkylene; and
R⁹ is alkyl;
each segment of the said polyurethane being contained in the proportion of x, y and z and being a block-type polymer, random type polymer, or a combination thereof.

The invention further provides a process for the preparation of polyurethanes (I) which comprises: (i) mixing an azobenzene monomer having two functional groups, which gives the structural unit B azo compound (B) with at least one of a polymer which gives the structural unit C (polymer (C)) and a diol compound which gives the structural unit D (diol compound (D)) in appropriate molar proportions, and then subjecting the resulting mixture to a polyaddition reaction with a diisocyanate which gives the structural unit A (diisocyanate (A)) in amounts equimolar with the sum of three preceding compounds; or (ii) reacting azo compound (B) and equimolecular diisocyanate (A), and at least one of (a) reacting polymer (C) and equimolecular diisocyanate (A), and (b) reacting diol compound (D) and equimolecular diisocyanate (A), and optionally mincing the resulting mixture of block copolymers thus obtained.

### Brief description of the drawings

Figure 1 is a graph showing the time-course of the release of a medicament in a composition coating with a polyurethane of the present invention, prepared in Example 13(c).

Figure 2 is a graph showing the time-course of the release of a medicament in a composition coated with a polyurethane of the present invention, prepared in Example 13(d).

Figure 3 is a graph showing the time-course of the release of a medicament in a composition coated with a polyurethane of the present invention, prepared in Example 14(d).

Figure 4 shows a time-course of a composition coated with a polyurethane of the present invention prepared in Example 16(d) following oral administration through the digestive organs and its disintegration.

Figure 5 is a graph showing a time-course of the concentration in blood, of a medicament in a composition coated with polyurethane of the present invention, prepared in Example 14(d) and 15(d) administered orally, and in a composition prepared in Reference Example 2.

In the present invention, unless otherwise specified, alkyl and alkylene groups are both straight-chained and branch-chained.

The bond to a benzene ring may be at any of o-, m-and p- positions.

The segmented polyurethanes (I) of the present invention consist of four repeat units, A to D, and comprise an azo segment linking A to B, i.e. A-B, a hydrophilic soft segment linking A to C, i.e. A-C and a hydrophobic hard segment linking A to D, i.e. A-D. When the proportion of each segments is represented by x, y and z, respectively, x, y and z are any numerical value in the range of 0.01 to 0.8, 0 to 0.80 and 0 to 0.99, respectively, and the sum of x, y and z is 1.0. The total molecular weight of the said polyurethane is about 1000-100000.

The segment A-B has an aromatic azo group which is reduced by intestinal bacteria and the polyurethanes are therefore degraded by the chemical change of this segment. The segment A-C is necessary for control of hydrophilicity of the polyurethanes. The segment increases the affinity of the polyurethane with intestinal bacteria and further provides water-solubility to a part of polyurethane to facilitate degradation of shaped products such as coatings. The segment A-D is a hydrophobic hard segment determining the physical property of the polyurethanes and controls the solubility of the polyurethane to solvents and its pharmacodynamics, and further greatly influences its strength and other characteristics, e.g. moldability of the composition.

R¹ in the structural unit A may be any divalent group known to those skilled in the art of high molecular compounds. Examples of such groups are aliphatic groups such as (CH₂)₄, (CH₂)₆, CH₂C(CH₃)₂CH₂CH₂CH(CH₃)CH₂, or (CH₂)₈, aromatic groups such as 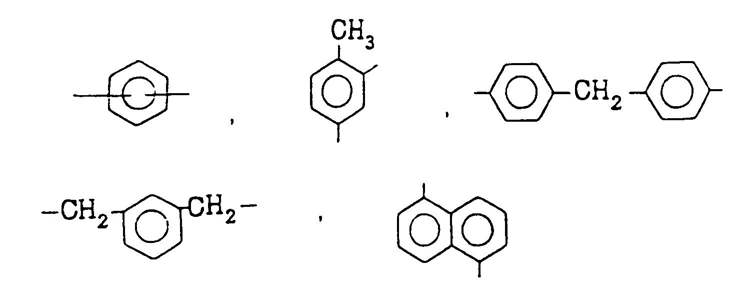
alicyclic groups such as 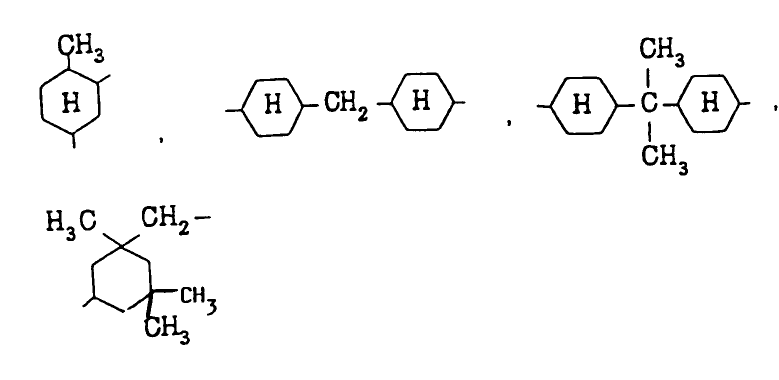
Examples of diisocyanates which give such groups are tetramethylene diisocyanate, hexamethylene diisocyanate, 2,2,5-trimehtylhexamethylene diisocyanate, octamethylene diisocyanate, o-, m- or p-phenylene diisocyanate, 2, 4- or 2,6-tolylene diisocyanate (abbreviated as TDI), 4,4′-diphenylmethane diisocyanate (abbreviated as MDI), o-, m- or p-xylylene diisocyanate, naphthalene 1,5-diisocyanate, hydrogenated TDI, hydrogenated MDI, dicyclohexyldimethylmethane 4,4′-diisocyanate, and isophorone diisocyanate. Further, precursors of diisocyanate in which an active diisocyanate is stabilized by a suitable blocking agent, may be used. Preferably R¹ is C₄₋₈ alkylene, isophorone, or a divalent group of a benzene of cyclohexyl, both which are optionally substituted by one to four methyl groups, and more preferably R¹ is 3,5,5-trimethylcyclohexan-l-yl-3-ylmethylene, hexamethylene or xylylene. When R¹ is a benzene or cyclohexyl group substituted by one to four methyl groups, it may be linked to the cyanate residues either directly or via a methyl group.

The azo-containing group represented by the structural unit B, may be a group having one azo group shown by (i) or an azo group shown by (ii). Y¹ and Y² are the same or different, and preferably the same. Y¹ and Y² may be attached to phenyl rings at the same or different positions. Examples of Y¹ and Y² are oxygen, imino, *CH₂O, OCH₂CH₂O and *OCH₂CH₂NH. Examples of R⁴ and R⁵ are hydrogen, chlorine, nitro and phenyl. Examples of azobenzene monomers having two functional groups, which give such groups are 4,4′-, 3,3′-, 3,4′-, 2,4′-, 2,2′- dihydroxyazobenzene, 1,4-bis[(4-hydroxyphenyl)azo]benzene, 4,4′-, 3,3′-, 2,2′-dihydroxymethylazobenzene, 4,4′-, 3,3′-, 2,2′-dihydroxyethylazobenzene, 4,4′-, 3,3′-, 2,2′-diaminoazobenzene, and derivatives thereof, e.g. diols such as 4,4′-bis(2-hydroxyethoxy)azobenzene, diamines such as 3,3′-bis(aminoacetylamino)azobenzene, and monoolamines such as 3-(3-hydroxypropyloxy)-4′-(4-aminopropylcarbamyl)azobenzene.

Monomers which contain a functional group at the o-position of an azo group are liable to be polymerized to give polyurethanes having relatively low molecular weight.

Peferably Y¹ and Y² are oxygen, imino, or a group of the formula: *R⁶-O or *R⁶-NH (in which R⁶ is C₁₋₄ alkylene), more preferably oxygen, imino or *CH₂O.

Preferably, the aza group is:

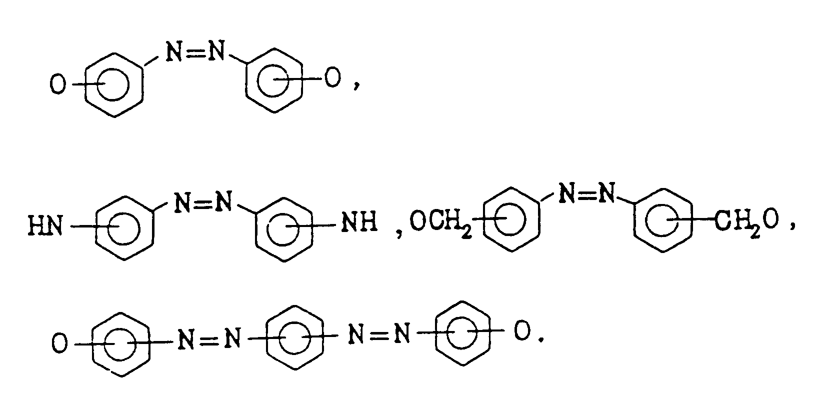

Examples of polyalkylene glycol residues represented by R² in structural unit C, are:
(CH₂CH₂O)ₚCH₂CH₂, 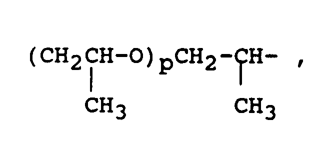
(CH₂CH₂CH₂CH₂O)ₚCH₂CH₂CH₂CH₂ ;
in which p is the degree of polymerization and is an integer from 1 to 500, preferably from 40 to 500. The two groups are the same, and are oxygen or imino, preferably oxygen.

Examples of polyalkylene glycol which give such groups are polyethylene glycol, polypropylene glycol, polytetramethylene glycol, poly(ethylenepropylene)glycol (copolymer of ethylene oxide and propylene oxide, having hydroxy groups at both ends) all being an average molecular weight of from 70 to 40000, preferably 1000 to 25000. Compounds having amino groups at both ends, corresponding to the said polyalkylene glycol may be effectively used.

Preferably the polyalkylene glycol is polymer of a C₁₋₄ alkylene glycol, more preferably polyethylene glycol.

Examples of R³ in structural unit D are
CH₂CH₂, 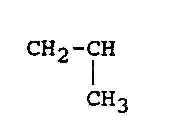
(CH₂)₄, CH₂CH₂OCH₂CH₂, 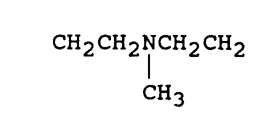
Examples of monomers which give such groups are aliphatic diols such as ethylene glycol, 1,2-propylene glycol, trimethylene glycol, tetramethylene glycol, diols having other functional group such as diethylene glycol, N-(2-hydroxyethyl)-N-methylethanolamine, and diols having an aromatic group. These diols may have other suitable substituents which do not react with the isocyanates. Preferably R³ is C₁₋₆ alkylene or a group of the formula: R⁷-O-R⁸ (in which R⁷ and R⁸ are C₁₋₆ alkylene), more preferably propylene, tetramethylene or 3-oxapentamethylene.

The polyurethanes (I) of the present invention can be prepared by methods known per se. That is, they are generally prepared by mixing an azobenzene monomer having two functional groups, which gives the structural unit B (merely referred to hereafter as azo compound (B)) with at least one of a polymer which gives the structural unit C (merely referred to hereafter as polymer (C)) and a diol compound which gives the structural unit D (merely referred to hereafter as diol compound (D)) in appropriate molar proportions, and then subjecting the mixture to a polyaddition reaction with a diisocyanate corresponding to the structural unit A (merely referred to hereafter as diisocyanate (A)) in equimolar amounts with the sum of the three preceding compounds. It is clear that the proportion of segments A-B, A-C and A-D, of the obtained polyurethanes, i.e., x, y and z depends on mole ratio of azo compound (B), polymer (C) and diol compound (D), used for the reaction. Usually the sequence of each segment is irregular. Polyurethanes of the present invention having block-type arrangement of sequence can be prepared by reacting azo compound (B) and equimolecular diisocyanate (A), followed by at least one of (a) reacting polymer (C) and equimolecular diisocyanate (A), and (b) reacting diol compound (D) and equimolecular diisocyanate (A). Further, the polyurethane having chains of short block-type arrangements of sequences can be prepared by performing the above separate polyaddition reactions followed by mincing steps. Any polyurethane described above can be degraded in the large intestine. Total molecular weight can be controlled by varying the sum of moles of the three kinds of compounds (B), (C) and (D) reacting with diisocyanate (A).

The said polyaddition reaction may be carried out in the presence of solvents or in bulk. An appropriate catalyst may be used to facilitate the reaction.

The characteristics and the degradation rate of the polyurethanes (I) are greatly changed by the proportion of each segment, x, y and z, which depend on mole ratio of azo compound (B), polymer (C) and diol compound (D) as starting materials. Generally speaking, the higher the proportion of the segment A-B or A-C, the greater the degradation rate by intestinal bacteria. Taking into account the degradation in the large intestine, the properties of the composition as a coating agent and the release of a medicament, a preferred proportion of each segment is as follows:
(i) in the polymer of the present invention comprising segments of A-B and A-C above, x : y is less than 0.6 : more than 0.4;
(ii) in compositions comprising segments of A-B and A-D above, x : z is less than 0.2 : more than 0.8; and
(iii) in compositions comprising segments of A-B, A-C and A-D, x : y : z is less than 0.2 : less than 0.1 : more than 0.7.

The molecular weight of polyurethanes (I) can be changed according to the required use. Preferable molecular weight of the polyurethane is about 8000 to 40000 relative to polystyrene standard, at the point of stability of the film. A polyurethane having low molecular weight used as a blending agent and may be mixed with other high molecular compounds to produce compositions may be used in coating medicaments and mixed films.

Furthermore, pharmaceutical adjuvants containing the polyurethane (I) as the main component are included in the present invention. The polyurethane (I) is degraded specifically in the large intestine, and therefore, it can be used as a pharmaceutical adjuvant for the purpose of delivering a medicament specifically to the large intestine at a high concentration. The term adjuvant described herein includes external coating agents for solid compositions for oral administration, such as tablets, granules, dispersible powders, pills and capsules, and carriers of sheet-like pharmaceutical compositions in which a medicament is filled.

Any medicament for the purpose of delivering specifically to the large intestine can be used without any restriction in the present invention. Examples of such medicaments are peptides such as insulin, vasopressin, interferons, and interleukins, guanidinobenzoic acid derivatives such as gabexate mesylate, camostat mesylate, and nafamostat mesylate, antiinflammatory agents such as aspirin, and thromboxane A₂ antagonists such as 9α,11α-dimethylmethano-13-aza-14-oxo-15β-hydroxy-15-cyclopentyl- 16,17,18,19,20-pentanor-11a-carbathromb-5Z-enoic acid.

When the polyurethanes of the present invention are used as external coating agents, they are dissolved or suspended in appropriate solvents (e.g. one or more of solvents selected from methylene chloride, chloroform, dioxane, acetone and ethanol) and then coated to a solid composition for oral administration separately prepared by pan-coating method or flow-coating method. If desired, water-insoluble polymers (e.g. ethyl cellulose or methacrylic acid coploymer) can be admixed besides the polyurethanes of the present invention.

When the polyurethanes of the present invention are used as a sheet-like pharmaceutical composition, they are dissolved or suspended into appropriate solvents (as above mentioned) with a medicament, and are molded into films by using casting methods known per se. The films thus obtained are crushed finely and are filled into capsules, etc. If desired, in preparing the films, water-insoluble polymers (e.g. ethyl cellulose, methacrylic acid copolymer,) and enteric polymer (e.g. hydroxypropylmethyl cellulose acetate succinate, carboxymethylethyl cellulose) can be also admixed.

By using the polyurethanes of the present invention as pharmaceutical adjuvants in which a medicament can be delivered specifically to the large intestine and at a high concentration, with minimal degradation and absorbtion in the small intestine, after oral administration. Consequently, it is possible to design pharmaceutical compositions with a reduced dose, fewer side effects and improved bio-availability over the prior art compositions.

The following Examples, Reference Examples and Experimental Example illustrate the present invention, but not limit the present invention.

### Example 1

A solution of 0.5 g (0.0023 mol) of 2,2′-dihydroxyazobenzene (o-DHAB) and 1.7 g (0.0224 mol) of propylene glycol (PG) in 100 ml of N,N′-dimethylformamide (DMF) was heated at 150°C with stirring under an stmosphere of nitrogen. When the reflux of DMF began, half of a solution of 5 g (0.030 mol) of hexamethylene diisocyanate (HMDI) in 50 ml of DMF was added dropwise all at once and vigorously stirred. The rest of the solution was added dropwise over three hours and further the mixture thus obtained was refluxed for one hour. Then the reaction mixture was cooled to room temperature, and poured into 1000 ml of diethyl ether. The resulting precipitate was filtered and dried in vacuo to give 3.3 g of the following product. yield : 46%

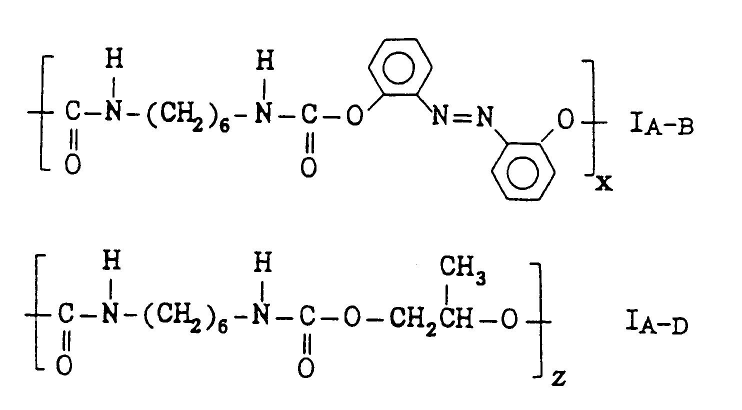

The product was confirmed to be polyurethane having segments of formulae I_{A-B} and I_{A-D} by measuring in ¹H-NMR spectrum using D₇-DMF as solvent. The proportion of x : z was 0.09 : 0.91. The average molecular weight of the product was 1300 (relative to polystylene standard, hereafter shown as the value relative to polystylene standard) by measuring a 1% tetrahydrofuran solution of the product by gel permeation chromatography (GPC).

### Example 2

A solution of 0.5 g (0.0023 mol) of o-DHAB and 1.7 g (0.224 mol) of PG in 20 ml of DMF was heated with stirring under an atmosphere of nitrogen. When a temperature of the mixture was at 100°C, half of a solution of 5 g (0.0265 mol) of m-xylylene diisocyanate (m-XDI) in 10 ml of DMF was added dropwise all at once and vigrously stirred. The rest of the solution was added dropwise over three hours and further the mixture thus obtained was refluxed for one hour. Then the reaction mixture was cooled to room temperature, and poured into 200 ml of diethyl ether. The resulting precipitate was filtered and dried in vacuo to give 6.6 g of the following product. yield : 92%

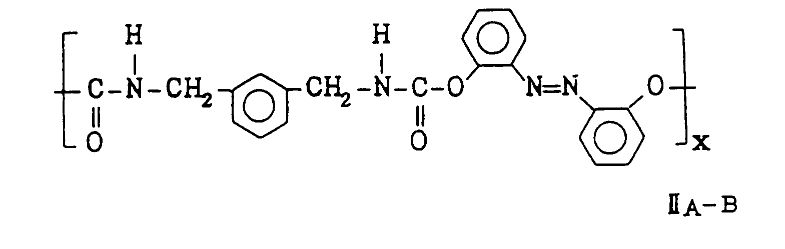

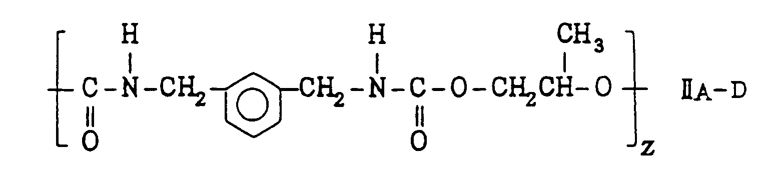

The product was confirmed to be polyurethane having segments of formulae II_{A-B} and II_{A-D} by measuring in the same manner as example hereinbefore described. The proportion of x : z was 0.09 : 0.91. The average molecular weight of the product was 1700 by measuring in the same manner as example hereinbefore described.

### Example 3

A solution of 0.5 g (0.0024 mol) of 4,4′-diaminoazobenzene and 5 g (0.0025 mol) of polyethylene glycol (average molecular weight 2000, PEG-2000) in 20 ml of 1,4-dioxane was heated with stirring under an atmosphere of nitrogen. Refluxing at ca. 100°C, a solution of 0.87 g (0.0049 mol) of m-XDI in 5 ml of 1,4-dioxane was added thereto dropwise over three hours, and the mixture was further refluxed for one hour. Then, the reaction mixture was cooled to room temperature, and poured into 200 ml of diethyl ether. The resulting precipitate was filtered and dried in vacuo to give 5.1 g of the following product. yield : 80% 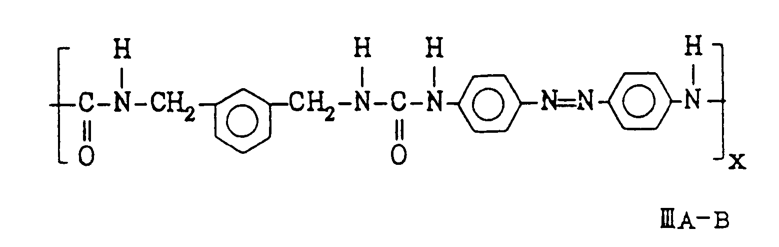 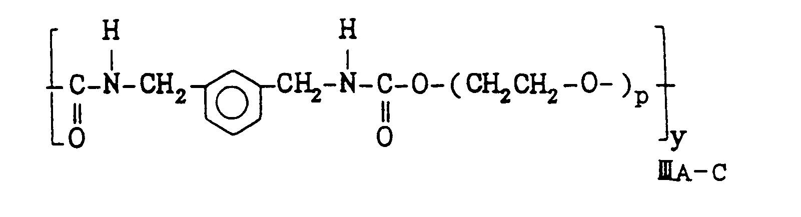
(wherein p is degree of polymerization)
The product was confirmed to be polyurethane having segments of formulae III_{A-B} and III_{A-C} by measuring in the same manner as examples hereinbefore described. The proportion of x : y = 0.49 : 0.5. The average molecular weight of the product was 4800 by measuring in the same manner as examples hereinbefore described.

### Example 4

A solution of 2.5 g (0.0329 mol) of PG and 2.5 g (0.0013 mol) of PEG-2000 in 20 ml of 1,4-dioxane was heated with stirring under an atmosphere of nitrogen. Refluxing at ca. 100°C, a solution of 0.25 g (0.0012 mol) of 4,4′-diaminoazobenzene in 5 ml of 1,4-dioxane and 9.6 g (0.051 mol) of m-XDI were added thereto dropwise at the same time over three hours, and the mixture was further refluxed for one hour. Then, the reaction mixture was cooled to room temperature, and poured into 300 ml of diethyl ether. The resulting precipitate was filtered and dried in vacuo to give 10.6 g of the following product. yield : 71% 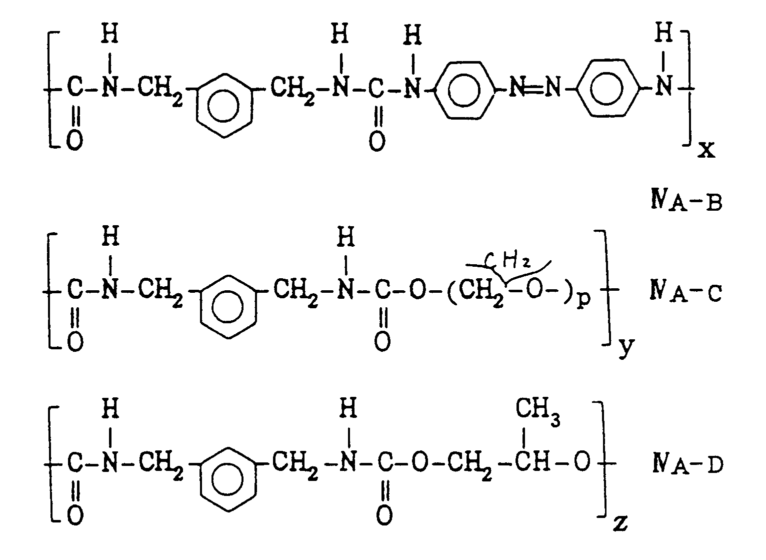
(wherein p is degree of polymesization)
The product was confirmed to be polyurethane having segments of formulae IV_{A-B}, IV_{A-C} and IV_{A-D} by measuring in the same manner as examples hereinbefore described. The proportion of x : y : z was 0.04 : 0.03 : 0.93. The average molecular weight of the product was 6400 by measuring in the same manner as examples hereinbefore described.

### Example 5

(a) A mixture of 1.05 g (0.0049 mol) of 3,3′-dihydroxyazobenzene (m-DHAB), 10 g (0.005 mol) of PEG-2000 and 10 g (0.131 mol) of PG was heated with stirring under an atmosphere of nitrogen. Half of 10 g (0.053 mol) of m-XDI was added dropwise all at once and vigorously stirred. The rest of m-XDI was added dropwise over three hours and further the mixture thus obtained was stirred for one hour at the same temperature to give reddish brown transparent solid. The solid was dissolved in 100 ml of 1,4-dioxane, and the solution was poured into 1000 ml of n-hexane. The resulting precipitate was filtered and dried in vacuo to give 28.6 g of the following product. yield : 92% 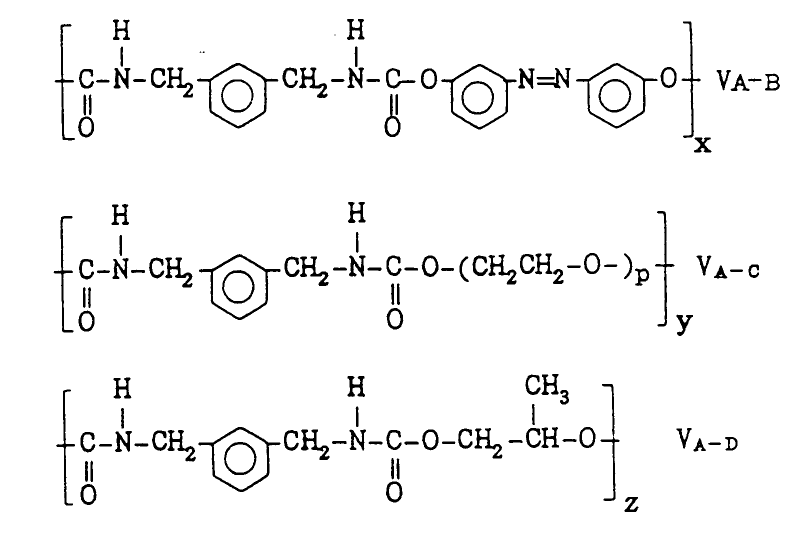 (wherein p is degree of polymerization)
   The product was confirmed to be polyurethane having segments of formulae V_{A-B}, V_{A-C} and V_{A-D} by measuring in the same manner as examples hereinbefore described. The proportion of x : y : z was 0.035 : 0.035 : 0.930. The average molecular weight of the product was 18000 by measuring in the same manner as examples hereinbefore described.
(b) 31.3 g of polyurethane of the present invention was prepared by using 1.69 g (0.0079 mol) of m-DHAB, 11 g (0.0055 mol) of PEG-2000 and 11 g (0.144 mol) of PG. The proportion of x : y : z was 0.050 : 0.035 : 0.915. The average molecular weight of the product was 16100. yield : 93%
(c) 15.9 g of polyurethane of the present invention was prepared by using one g (0.0047 mol) of m-DHAB, 3.1 g (0.0016 mol) of PEG-2000 and 3.1 g (0.0407 mol) of PG. The proportion of x : y : z was 0.1 : 0.03 : 0.87. The average molecular weight of the product was 18100. yield : 92%

### Example 6

A mixture of 1.6 g (0.0075 mol) of m-DHAB, 14 g (0.0007 mol) of polyethylene glycol (average molecular weight 20000, PEG-20000) and 6 g (0.0667 mol) of 1,4-butanediol was heated with stirring under an atmosphere of nitrogen. 15 g (0.0797 mol) of m-XDI was added dropwise over two hours and further the mixture thus obtained was stirred for 30 minutes at the same temperature to give 33 g of reddish brown solid. yield : 90 % 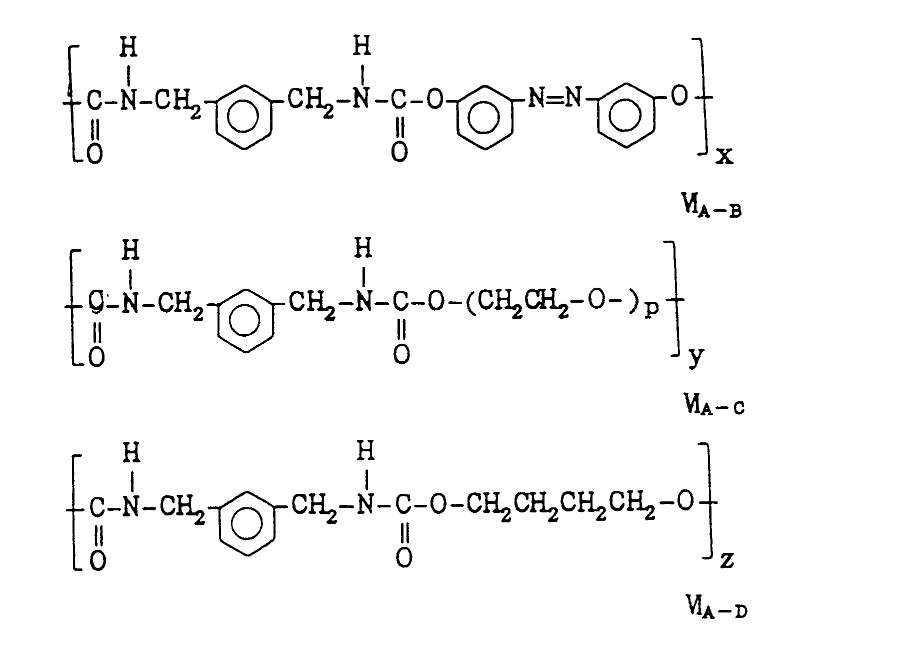
(wherein p is degree of polymerization)
The product was confirmed to be polyurethane having segments of formulae VI_{A-B}, VI_{A-C} and VI_{A-D} by measuring in the same manner as examples hereinbefore described. The proportion of x : y : z was 0.10 : 0.01 : 0.89. The average molecular weight of the product was 30000 by measuring in the same manner as examples hereinbefore described.

Further, the derivative of formula (VI) was prepared by using polyethylene glycol (average molecular weight 8300) as C component in the same manner.

### Example 7

A mixture of 0.945 g (0.0044 mol) of m-DHAB, 4 g (0.002 mol) of PEG-2000 and 4 g (0.0377 g) of diethylene glycol was heated with stirring under an atmosphere of nitrogen. 8.3 g (0.0441 mol) of m-XDI was added dropwise thereto over 30 minutes at 110°C and the mixture was stirred for three hours at the same temperature to give reddish brown solid. The solid was dissolved in 100 ml of 1,4-dioxane, and the solution was poured into 1000 ml of n-hexane. The resulting precipitate was filtered and dried in vacuo to give 15.0 g of the product. yield : 87% 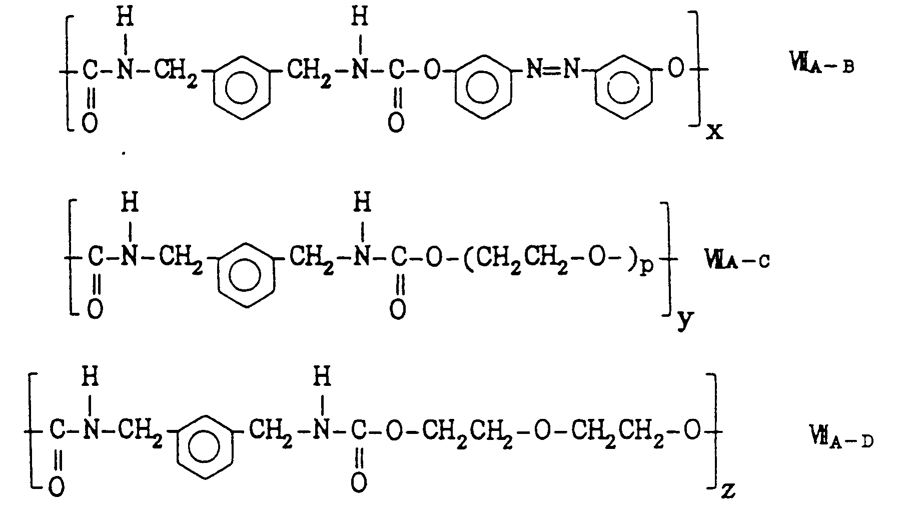
(wherein p is degree of polymerization)
The product was confirmed to be polyurethane having segments of formulae VII_{A-B}, VII_{A-C} and VII_{A-D} by measuring in the same manner as examples hereinbefore described. The proportion of x : y : z was 0.10 : 0.05 : 0.85. The average molecular weight of the product was 20000 by measuring in the same manner as examples hereinbefore described.

### Example 8

A mixture of 0.118 g (0.00055 mol) of m-DHAB, 0.5 g (0.00025 mol) of PEG-2000 and 0.5 g (0.00471 mol) of diethylene glycol was heated with stirring under an atmosphere of nitrogen. After rising a temperature till 65°C to give heterogeneous solution, 0.927 g (0.00551 mol) of HMDI was added thereto. After rising a temperature of the mixture gradually with vigorously stirring, the mixture was stirred at 120°C for 6 hours. The resulting solid was dissolved in 20 ml of 1,4-dioxane and the solution was poured into 200 ml of n-hexane. The resulting precipitate was filtered and dried in vacuo to give 1.98 g of the product. yield : 97% 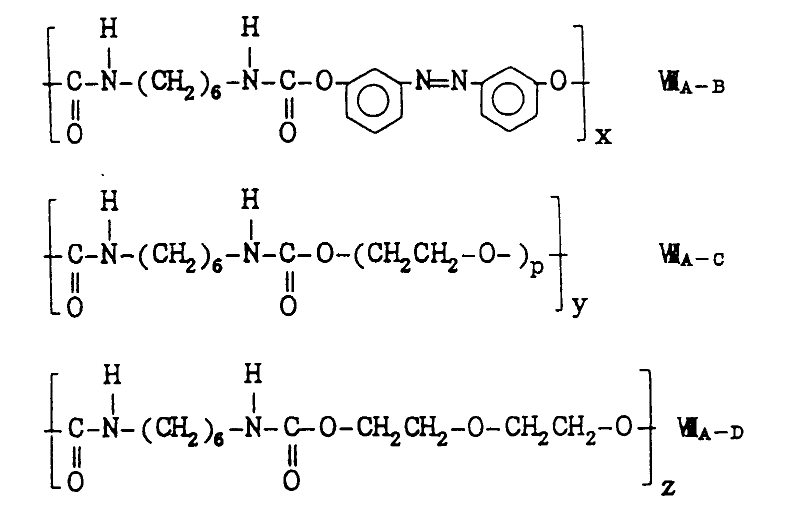
(wherein p is degree of polymerization)
The product was confirmed to be polyurethane having segments of formulae VIII_{A-B}, VIII_{A-C} and VIII_{A-D} by measuring in the same manner as examples hereinbefore described. The proportion of x : y : z was 0.1: 0.045 : 0.855. The average molecular weight of the product was 20000 by measuring in the same manner as example hereinbefore described.

### Example 9

A mixture of 1.28 g (0.0060 mol) of m-DHAB, 4 g (0.002 mol) of PEG-2000 and 4 g (0.0526 mol) of PG was heated with stirring under an atmosphere of nitrogen. 10.26 g (0.061 mol) of HMDI was added dropwise thereto over three hours and the mixture was stirred for one hour at the same temperature to give reddish brown transparent solid. The solid was dissolved in 200 ml of a mixture of ethanol and acetone (1 : 1), and the solution was poured into 2000 ml of n-hexane. The resulting precipitate was filtered and dried in vacuo to give 18.1 g of the product. yield : 98% 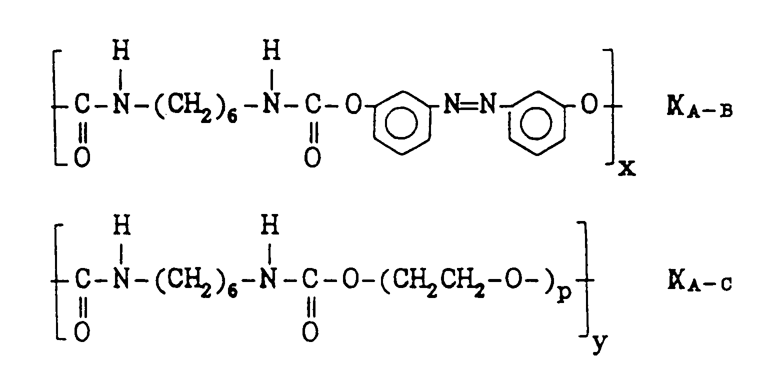 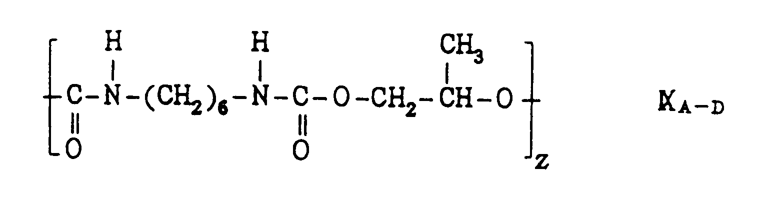
(wherein p is degree of polymerization)
The product was confirmed to be polyurethane having segments of formulae IX_{A-B}, IX_{A-C} and IX_{A-D} by measuring in the same manner as examples hereinbefore described. The proportion of x : y : z was 0.1 : 0.03 : 0.87. The average molecular weight of the product was 14000 by measuring in the same manner as examples hereinbefore described.

Several derivatives of having each segments of formula IX in various proportion were prepared in the same manner as above by using various amounts of m-DHAB, PEG-2000 and PG as starting materials. The average molecular weight of the products was in the range of 11000-14000. The polyurethane wherein the proportion of x : y : z is 0.10 : 0.074 : 0.826 was obtained by using 1.029 g (0.0048 mol) of m-DHAB, 7 g (0.0035 mol) of PEG-2000 and 3 g (0.0394 mol) of PG as starting materials. The polyurethane wherein the proportion of x : y : z is 0.10 : 0.015 : 0.885 was obtained by using 1.114 g (0.0052 mol) of m-DHAB, 1.5 g (0.0008 mol) of PEG-2000 and 3.5 g (0.0460 mol) of PG as starting materials.

### Example 10

A mixture of 3.63 g (0.015 mol) of 3,3′-dihydroxymethylazobenzene (m-DHMAB), 10 g (0.005 mol) of PEG-2000 and 10 g (0.132 mol) of PG was heated with stirring under an atmosphere of nitrogen. 25.4 g (0.151 mol) of HMDI was added dropwise thereto over three hours at 110°C and the mixture was stirred for one hour at the same temperature to give red transparent solid. The solid was dissolved in 300 ml of ethanol and the solution was poured into 3000 ml of n-hexane. The resulting precipitate was filtered and dried in vacuo to give 48.2 g of the product. yield : 98% 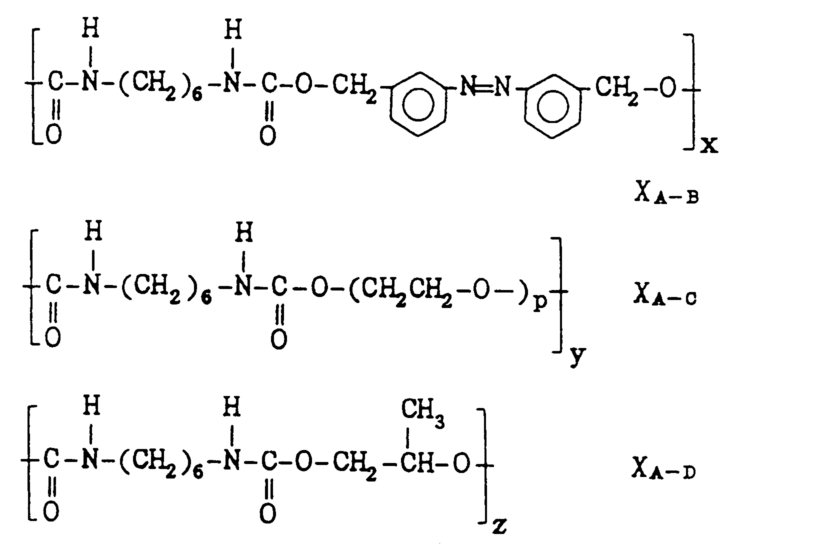
(wherein p is degree of polymerization)
The product was confirmed to be polyurethane having segments of formulae X_{A-B}, X_{A-C} and X_{A-D} by analyzing in the same manner as examples hereinbefore described. The proportion of x : y : z was 0.10 : 0.03 : 0.87. The average molecular weight of the product was 43000 by measuring in the same manner as examples hereinbefore described.

In the same manner as above, the polyurethane wherein the proportion of x : y : z is 0.10 : 0.01 : 0.89 and the average molecular weight is 15000 , was obtained by using 5.08 g (0.021 mol) of m-DHMAB, 6 g (0.003 mol) of PEG-2000 and 14 g (0.184 mol) of PG as starting materials, and the polyethylene wherein the proportion of x : y : z is 0.10 : 0.07 : 0.83 and the average molecular weight is 16700 was obtained by using 2.32 g (0.0096 mol) of m-DHMAB, 14 g (0.007 mol) of PEG-2000 and 6 g (0.079 mol) of PG as starting mterials.

### Example 11

A solution of 0.73 g (0.0023 mol) of 1,4-bis[(4-hydroxyphenyl)azo]benzene and 1.7 g (0.0224 mol) of PG in 100 ml of DMF was heated with stirring to 150°C under an atmosphere of nitrogen. When the reflux of DMF began, half of a solution of 5 g (0.030 mol) of HMDI in 50 ml of DMF was added dropwise all at once and the solution thus obtained was vigorously stirred. The rest of the solution of HMDI was added over three hours and further the mixture was refluxed for one hour. Then the reaction mixture was cooled to room temperature, and poured into 1000 ml of diethyl ether. The resulting precipitate was filtered and dried in vacuo to give 3.5 g of the product. yield : 46%

The product was confirmed to be polyurethane having segments of formulae XI_{A-B} and XI_{A-D} by measuring in the same manner as examples hereinbefore described. The proportion of x : z was 0.09 : 0.91. The average molecular weight of the product was 15000 by measuring in the same manner as examples hereinbefore described.

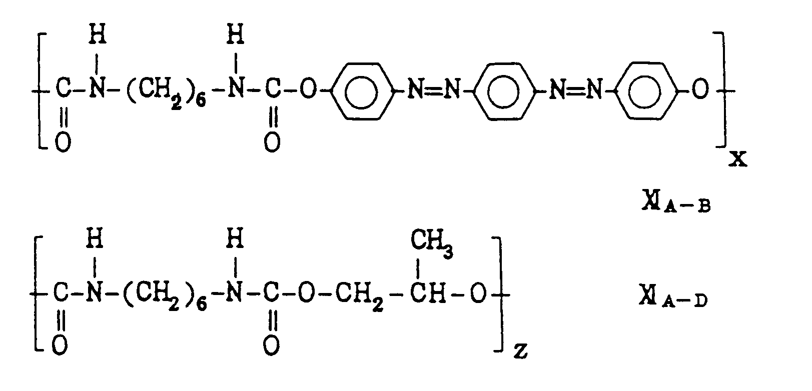

### EXAMPLE 12

A mixture of 7.312g (0.0302 mol) of m-DHMAB, 20g (0.010 mol) of PEG-2000 and 20 g (0.262 mol) of PG was heated with stirring under an atmosphere of nitrogen. 67.132g (0.302 mol) of isophorone diisocyanate was added dropwise thereto over three hours at 110°C and the mixture was stirred for one hour at the same temperature to give a red transparent solid. The solid was dissolved in 300 ml of ethanol and the solution was poured into 3000 ml of diethyl ether. The resulting precipitate was filtered and dried in vacuo to give 87.131g of the product. yield: 76%

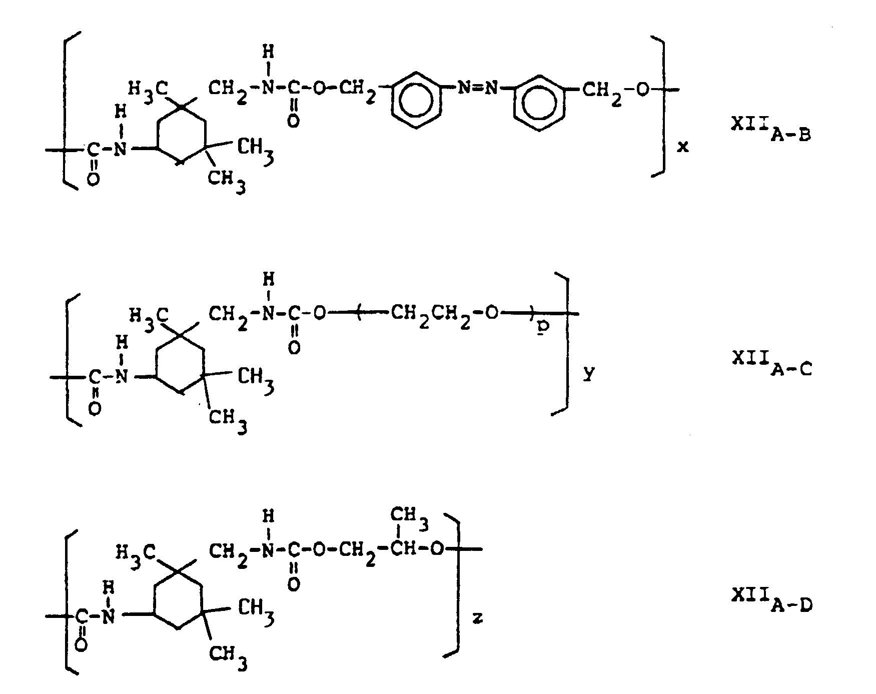

The product was confirmed to be polyurethane having the segments of formulae XII_{A-B}, XII_{A-C} and XII_{A-D} by measuring in the same manner as examples hereinbefore described. The proportion of x:y:z was 0.10:0.03:0.87. The average molecular weight of the product was 10100 by measuring in the same manner as examples hereinbefore described.

### Reference Example 1

5 parts in weight of p-(p-guanidinobenzoyloxy)phenylacetic acid N,N-dimethylcarbamoylmethyl ester methanesulfonate, 3 parts in weight of lactose, 1 part in weight of microcrystalline cellulose (Avicel® PH 101, prepared by Asahi Kasei Co.) and 1 part in weight of low-substituted hydroxypropyl cellulose (L-HPC, prepared by Shin-etsu Kagaku Co.) were admixed, and then granules of purified sugar (Nonparel® 103, Freund Ind. Ltd. Co.) as core were powder-coated with the mixture obtained by using a 5% aqueous solution of hydroxypropyl cellulose (HPC-L, prepared by Shin-etsu Kagaku Co.) as binder, by centrifuged flow granulator (CF granulator, Freund Inc. Ltd. Co.) by the method known per se to obtain each pellets of about 1, 2 and 3 mm diameter.

### Example 13

The pellets prepared in Reference Example 1 were coated with a 5% solution of the polyurethane of the present invention, prepared in Example 1, in a mixture of acetone and ethanol (1 : 1) by pan-coating method to obtain pharmaceutical compositions degraded in the large intestine, of the present invention. Pharmaceutical compositions of the present invention were obtained in the same manner as above by using polyurethanes and solvents as shown in the following Table I.

**Table I**

| Example No. | used polyurethane | solvents |
|---|---|---|
| 13 (a) | Example 3 | chloroform/dioxane = 1/1 |
| 13 (b) | Example 5 (a) | chloroform/dioxane = 1/1 |
| 13 (c) | Example 5 (b) | chloroform/ethanol = 1/1 |
| 13 (d) | Example 5 (c) | methylene chloride/ethanol = 1/1 |
| 13 (e) | Example 10 | acetone/ethanol = 1/1 |

### Reference Example 2

One part in weight of 9α,11α-dimethylmethano-13-aza-14-oxo-15β-hydroxy-15-cyclopentyl-16,17,18,19,20-pentanor-11a-carbathromb-5Z-enoic acid N-methyl-D-glucamine salt (abbreviated as Compound A hereafter), 5 parts in weight of lactose, 2 parts in weight of microcrystalline cellulose and 2 parts in weight of low-substituted hydroxypropyl cellulose were admixed, and then granules of purified sugar as core were powder-coated with the mixture obtained by using a 5% aqueous solution of hydroxypropyl cellulose as binder, by centrifuged flow granulator by the method known per se to obtain three kind of pellets of about 1, 2 and 3 mm diameter.

### Example 14

The pellets prepared in Reference Example 2 were coated with a 5% solution of the polyurethane of the present invention, prepared in Example 1, in a mixture of acetone and ethanol (1 : 1) by pan-coating method to obtain pharmaceutical compositions degraded in the large intestine, of the present invention. Pharmaceutical compositions of the present invention were obtained in the same manner as above by using polyurethanes and solvents as shown in the following Table II.

**Table II**

| Example No. | used polyurethane | solvents |
|---|---|---|
| 14 (a) | Example 3 | chloroform/dioxane = 1/1 |
| 14 (b) | Example 5 (a) | chloroform/dioxane = 1/1 |
| 14 (c) | Example 5 (b) | chloroform/ethanol = 1/1 |
| 14 (d) | Example 5 (c) | methylene chloride/ethanol = 1/1 |
| 14 (e) | Example 10 | acetone/ethanol = 1/1 |

### Reference Example 3

250 g of Compound A, 740 g of glycerin tricaprylate (Panacete® 800, prepared by Nippon Yushi Co.) and 10 g of bean lecithin (prepared by Hohnen Seiyu Co.) were admixed enough with stirring. The obtained suspension was sieved through a 100-mesh sieve and then was filled into soft capsules with a rotary-capsule machine by methods known per se to obtain about 2000 soft capsules.

### Example 15

The soft capsules prepared in Reference Example 3 were coated with a 5% solution of the polyurethane of the present invention, prepared in Example 1, in a mixture of acetone and ethanol (2 : 8) by pan-coating method to obtain pharmaceutical compositions degraded in the large intestine, of the present invention. Pharmaceutical compositions of the present invention were obtained in the same manner as above by using polyurethanes and solvents as shown in the following Table III

**Table III**

| Example No. | used polyurethane | solvents |
|---|---|---|
| 15 (a) | Example 3 | chloroform/dioxane = 1/1 |
| 15 (b) | Example 5 (a) | chloroform/dioxane = 1/1 |
| 15 (c) | Example 5 (b) | chloroform/ethanol = 1/1 |
| 15 (d) | Example 5 (c) | methylene chloride/ethanol = 1/1 |
| 15 (e) | example 10 | acetone/ethanol = 1/1 |

### Reference Example 4

Three kind of pellets of about 1, 2 and 3 mm diameter were obtained by using 5 parts in weight of barium sulfate, 4 parts in weight of lactose, 1 part in weight of microcrystalline cellulose and 4 parts in weight of low-substituted hydroxypropyl cellulose, in the same manner as Reference Example 1.

### Example 16

The pellets prepared in Reference Example 4 were coated with a 5% solution of the polyurethane of the present invention, prepared in Example 1, in a mixture of ethanol and acetone (8 : 2) by pan-coating method to obtain pharmaceutical compositions degraded in the large intestine, of the present invention. Pharmaceutical compositions of the present invention were obtained in the same manner as above by using polyurethanes and solvents as shown in the following Table IV.

**Table IV**

| Example No. | used polyurethane | solvents |
|---|---|---|
| 16 (a) | Example 3 | chloroform/dioxane = 1/1 |
| 16 (b) | Example 5 (a) | chloroform/dioxane = 1/1 |
| 16 (c) | Example 5 (b) | chloroform/ethanol = 1/1 |
| 16 (d) | Example 5 (c) | methylene chloride/ethanol 1/1 |
| 16 (e) | Example 10 | acetone/ethanol = 1/1 |

### Experimental Example 1

### Degradation of the polyurethane of the present invention

### Method

Experiment was carried out by applying human feces-incubation method as described by T. Mitsuoka et. al., (Kansensho-Gakkai Zasshi, 45(9), 406-419, 1981).

That is, the semi-solid general anaerobic medium (GAM, Nissui Ltd. Co.) was added to approximately one gram of human feces to obtain the mixture of 10 g in weight. The mixture further diluted with GAM to 10 times to obtain the 10⁻² fold medium. The medium thus obtained was incubated in approximately 200 fold GAM at 37°C for two days anaerobically to obtain the test medium.

After the polyurethane films of the present invention were incubated in 20 ml of the test medium for two days, the degree of degradation of the films by intestinal flora was investigated by measure tensile strength,tensile elongation and molecular weight. Similarly the experiment was carried out in the test medium not including human feces as control. The result was shown in the following Table V.

**Table V**

| Example No. of polyurethane | kind of test medium | average molecular weight | tensile strength (MPa) | elongation (%) |
|---|---|---|---|---|
| 3 | (A) | 4800 | <30 | 250 |
| | (B) | 4700 | <2 | 3 |
| 5(b) | (A) | 16100 | <122 | 540 |
| | (B) | 17600 | <31 | 17 |
| 5(c) | (A) | 18100 | 1) | 1) |
| | (B) | 9600 | 2) | 2) |
| 11 | (A) | 15000 | <55 | 450 |
| | (B) | 13000 | <7 | 12 |
| (A) means test medium not including human feces. (B) means test medium including human feces. 1) It was impossible to measure because the films absorbed water to swell. It was confirmed by electron microscope that the surface of the film was smooth and the film was not degraded. 2) It was impossible to measure because of severe degradation of the film. | | | | |

### Consideration

It is understood that the tensile strength and elongation, of the polyurethane films of the present invention are remarkably reduced by the incubation thereof in test medium including human feces, compared with the incubation in test medium not including human feces. Further, the molecular weight of the polyurethane tends to lower.

It is considered from the fact, that azo bonds in the polyurethane were chemically altered by the action of intestinal bacteria living in human feces and thereby the structure of the film is altered and the tensile strength and elongation thereof are reduced.

### Experimental Example 2

### Release of a medicament in composition coated with polyurethane of the present invention

### Method

The compositions prepared in Example 13 (c), 13 (d) and 14 (d) were incubated in mediums including or not including human feces, used in Experimental Example 1. The medium was withdrawn after given periods of time and the medicament released in the medium was measured by high-performance liquid chromatography.

### Result

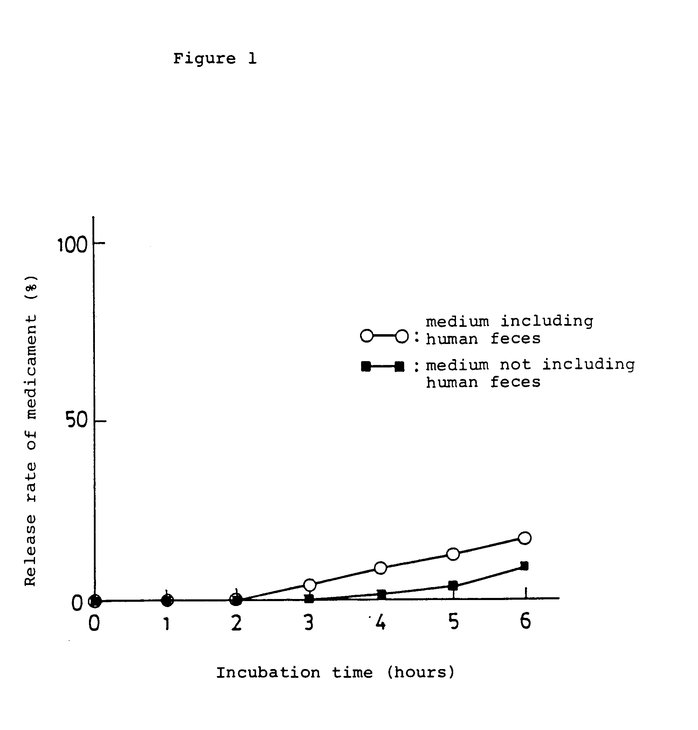

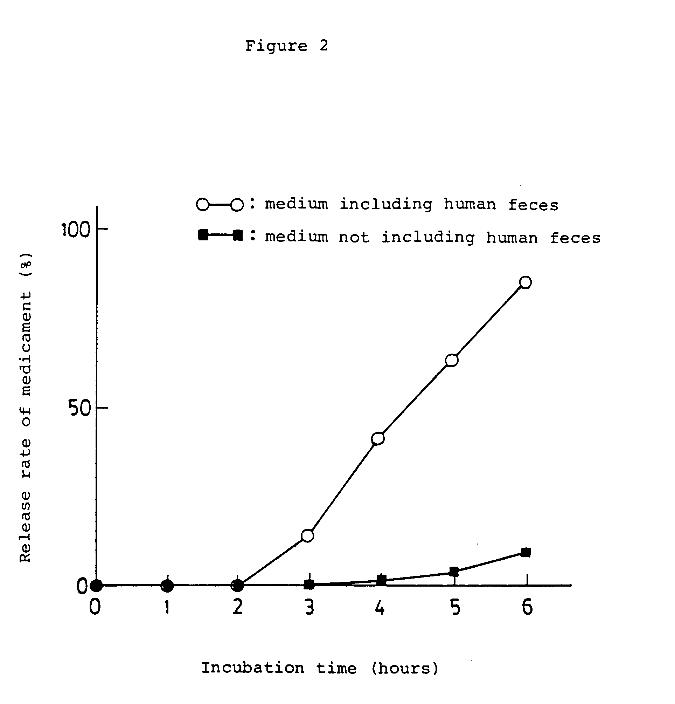

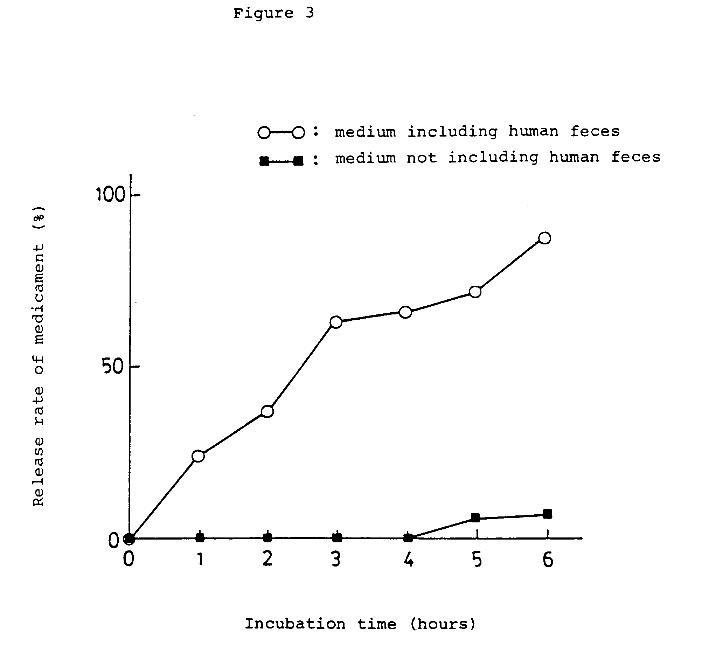

Result was shown in Figure 1, 2 and 3.

### Consideration

The release rate of a medicament in the composition of the present invention incubated in a medium including human feces was highly sperior to that incubated in a medium not including human feces.

Furthermore, it is considered that the compositions having various release rate of a medicament are obtained according to the proportion x of segment containing aromatic azo groups.

### Experimental Example 3

### Disintegration of a medicament in composition coated with polyurethane of the present invention in oral administration

### Method

Compositions prepared in Example 16 (d) (each 25 pellets ) were administered orally with 50 ml of water in 14 beagle dogs fasting overnight. After given periods of time (2 dogs after 2 hours, 2 dogs after 3 hours, 6 dogs after 4 hours and 4 dogs after 5 hours), the dogs were anesthetized intravenously by sodium pentobarbital, and then photographed by X rays to trace the compositions in digestive organs.

### Result

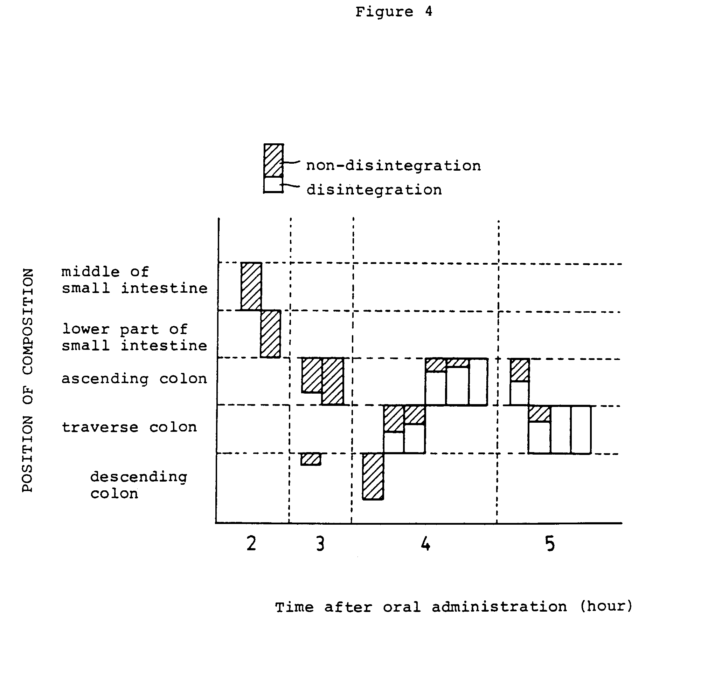

The result is shown in Figure 4. In figure, each oblong represents a total volume of pellets administered and represents the position of pellets after given periods of times. Opened and shaded parts in oblong indicates the proportion of disintegration.

### Consideration

It is confirmed that the compositions coated with polyurethane of the present invention reached colon after 3 hours from administration, and most of them were disintegrated in ascending and traverse colon after 4 hours, to release a medicament.

### Experimental Example 4

### Absorption of a medicament in composition coated with polyurethane of the present invention in oral administration

### Method

Compositions prepared in Example 14 (d) and 15 (d) were administered orally with 50 ml of water in beagle dogs fasting overnight. After given periods of time, the concentration of a medicament in blood was measured by high performance liquid chromatography.

### Result

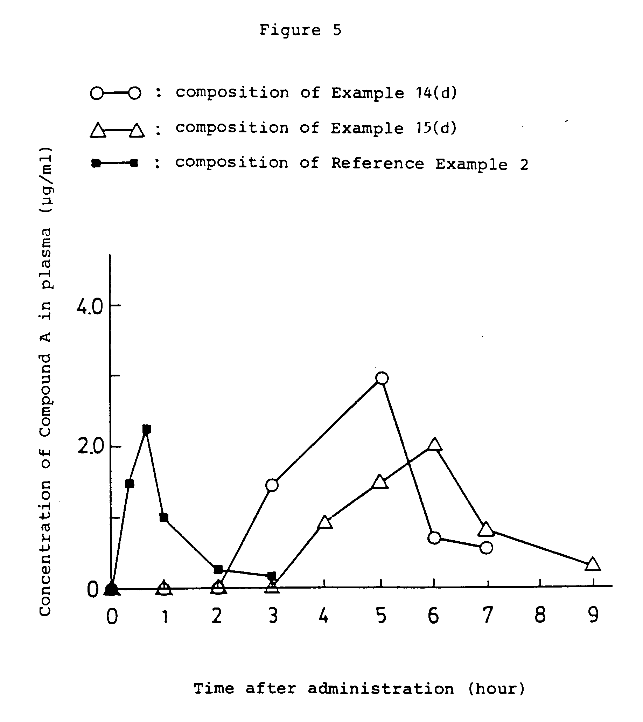

The result was shown in figure 5.

### Consideration

Taking the result of Experimental Example 3 into consideration that it takes a medicament about 3 hours to reach ascending colon from mouth, the result of the present experiment (that is, a medicament is released in blood after 2 to 3 hours from administration) is reasonable. From the result, it is considered that the composition coated with polyurethanes of the present invention is ideal composition which is not disintegrated before it reaches colon, and is first degraded in colon by the action of intestinal bacteria to release medicament.

## Claims (Claims for the following Contracting State(s): AT, BE, CH, DE, DK, FR, GB, IT, LI, LU, NL, SE)

1. A polyurethane (I) of 1000 to 100000 in average molecular weight, comprising plural segments, which are each structural units of A-B, A-C and A-D of the formulae: 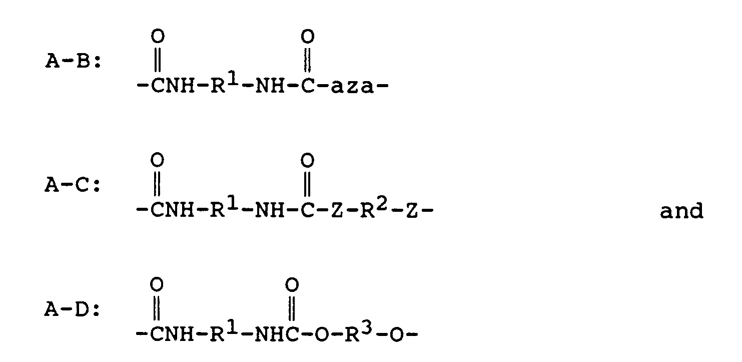 the molar proportion of the said segments of A-B, A-C and A-D i.e. x : y : z being 0.01 to 0.8 : O to 0.80 : 0 to 0.99, provided that the sum of x, y and z is 1.0; the segments A, B, C and D being structural units of the formulae: 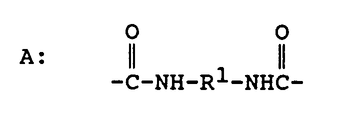
B: -aza-
C: -Z-R²-Z- and
D: -O-R³-O-;
wherein R¹ is a skeleton of a diisocyanate and the three R¹ groups in each of the segments A-B, A-C and A-D are same or different;
aza is a group of the formula: 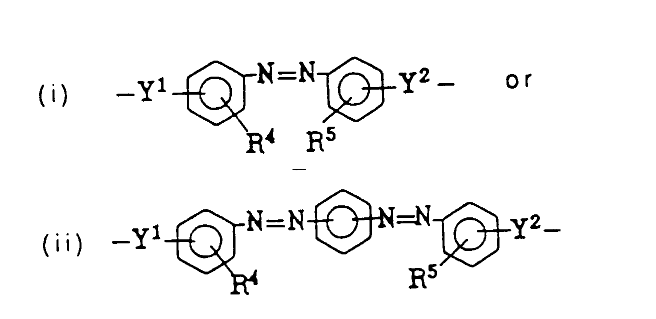 wherein Y¹ and Y², which may be the same or different, represent oxygen, imino (-NH-) or a group of the formula:
*R⁶-O, *R⁶-NH, O-R⁶-O, *NHCO-R⁶-NH or *CONH-R⁶-NH;
wherein R⁶ is alkylene and the atom or the end of the group marked by * is bonded to the phenyl ring;
R⁴ and R⁵ are each, independently, hydrogen, halogen, nitro or phenyl;
R² is a polyalkylene glycol residue;
Z is oxygen or imino and both groups Z are the same;
R³ is alkylene or a group of the formula:
R⁷-O-R⁸, R⁷-NH-R⁸ or 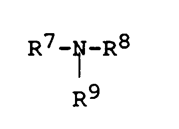 wherein R⁷ and R⁸, which may be the same or different, are alkylene; and
R⁹ is alkyl;
each segment of the said polyurethane being contained in the proportion of x, y and z and being a block-type polymer, random type polymer, or a combination thereof.

2. A polyurethane according to claim 1 in which R¹ is a C₂₋₉ alkylene, an aromatic group of the formula: 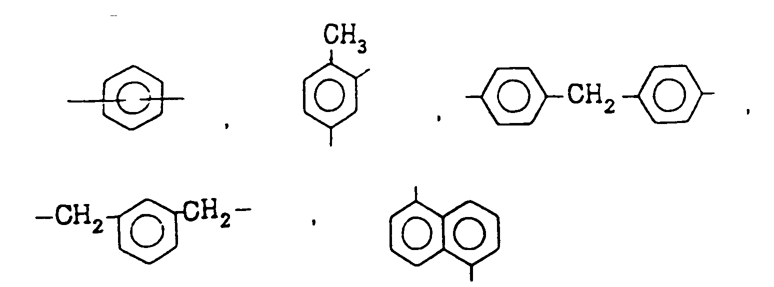 or an alicylic groups of the formula:

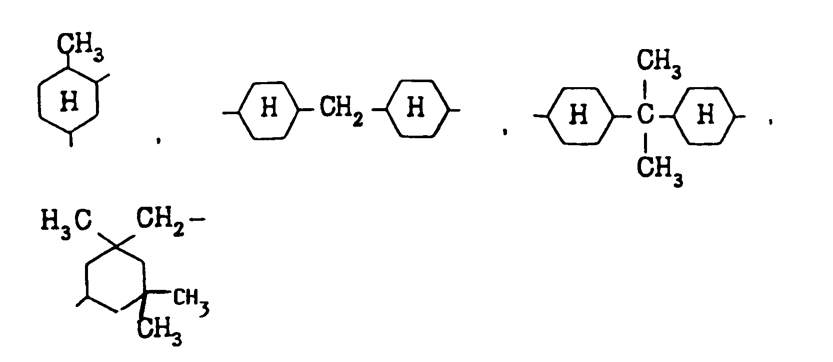

3. A polyurethane composition according to claim 1 or 2 in which the diisocyanate residue, unit A, is the residue of tetramethylene diisocyanate, hexamethylene diisocyanate, 2,2,5-trimethylhexamethylene diisocyanate, octamethylene diisocyanate, o-, m- or p-phenylene diisocyanate, 2,4- or 2,6-tolylene diisocyanate (TDI), 4,4′-diphenylmethane diisocyanate (MDI), o-, m- or p-xylylene diisocyanate, naphthalene 1,5-diisocyanate, hydrogenated TDI, hydrogenated MDI, dicyclohexyldimethylmethane 4,4′-diisocyanate, or isophorone diisocyanate.

4. A polyurethane according to claim 1 or 2, wherein R¹ is C4-8 alkylene, or a divalent group of a benzene or cyclohexyl, both which may be optionally substituted by one to four methyl groups, and R¹ may be linked to the cyanate residues either directly, or when present, optionally via a methyl group.

5. A polyurethane according to claim 4 wherein R¹ is hexamethylene, 3,5,5-trimethylcyclohexan-1-yl-3-ylmethylene, or xylylene.

6. A polyurethane according to any one of the preceding claims wherein aza is a group of the formula: 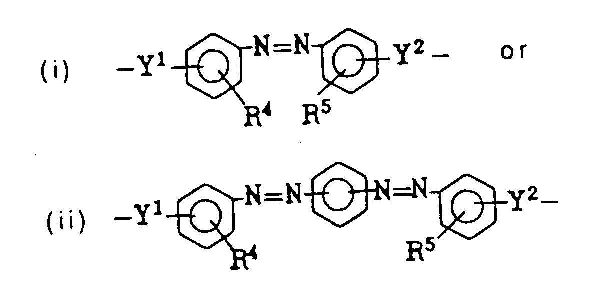 wherein Y¹ and Y², which may be the same or different, are oxygen, imino or a group of the formula: *R⁶-O or *R⁶-NH (in which R⁶ is C1-4 alkylene) and R⁴ and R⁵ are hydrogen.

7. A polyurethane according to any one of the preceding claims wherein R² is a polymer residue of C1-4 alkylene glycol.

8. A polyurethane according to any one of the preceding claims wherein R³ is C1-6 alkylene or a group of the formula: R⁷-O-R⁸ (in which R⁷ and R⁸, which may be the same or different, are C1-6 alkylene).

9. A polyurethane according to any one of the preceding claims wherein proportion of the segments, x : y is less than 0.6: more than 0.4, x : z is less than 0.2 : more than 0.8, or x : y : z is less than 0.2 : less than 0.1 : more than 0.7.

10. A polyurethane according to claim 1, wherein R¹ is a group of the formula: 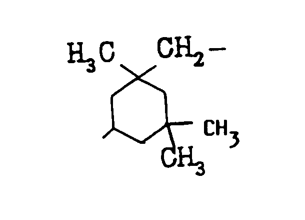 and R¹ in each of the segments A-B, A-C and A-D are the same,
aza is a group of the formula: 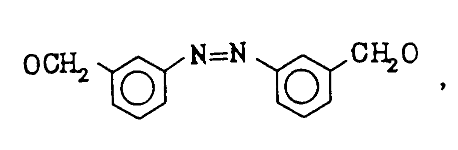 Z-R²-Z is a polyethylene glycol residue and R³ is 1,2-propylene.

11. A process for the preparation of the polyurethane (I) as claimed in claim 1, which comprises: (i) mixing an azobenzene monomer having two functional groups, which gives the structural unit B (azo compound (B)) with at least one of a polymer which gives the structural unit C (polymer (C)) and a diol compound which gives the structural unit D (diol compound (D)) in appropriate molar proportions, and then subjecting the resulting mixture to a polyaddition reaction with a diisocyanate which gives the structural unit A (diisocyanate (A)) in amounts equimolar with the sum of three preceding compounds; or (ii) reacting azo compound (B) and equimolecular diisocyanate (A), and at least one of (a) reacting polymer (C) and equimolecular diisocyanate (A), and (b) reacting diol compound (D) and equimolecular diisocyanate (A), and optionally mincing the resulting mixture of block copolymers thus obtained.

12. A pharmaceutical adjuvant containing a polyurethane claimed in any one of claims 1 to 10.

## Claims (Claims for the following Contracting State(s): ES, GR)

1. A process for the preparation of polyurethanes (I) of 1000 to 100000 in molecular weight, comprising plural segments, which are each structural units of A-B, A-C and A-D of the formulae: 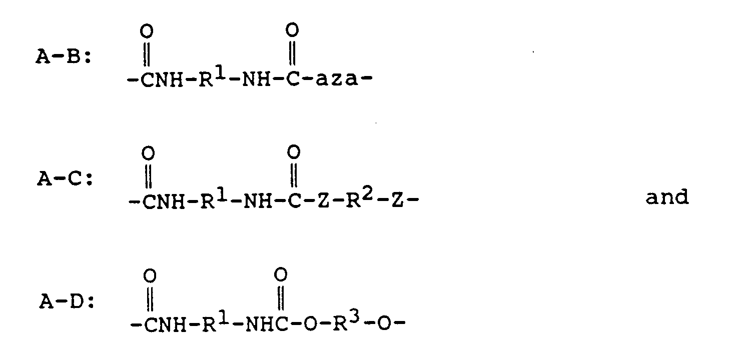 the molar proportion of the said segments of A-B, A-C and A-D i.e. x : y : z being 0.01 to 0.8 : O to 0.80 : 0 to 0.99, provided that the sum of x, y and z is 1.0; the segments A, B, C and D being structural units of the formulae: 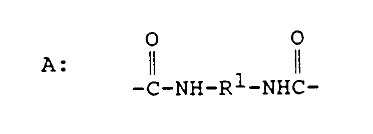
B: -aza-
C: -Z-R²-Z- and
D: -O-R³-O-;
wherein R¹ is a skeleton of a diisocyanate and the three R¹ groups in each of the segments A-B, A-C and A-D are same or different;
aza is a group of the formula: 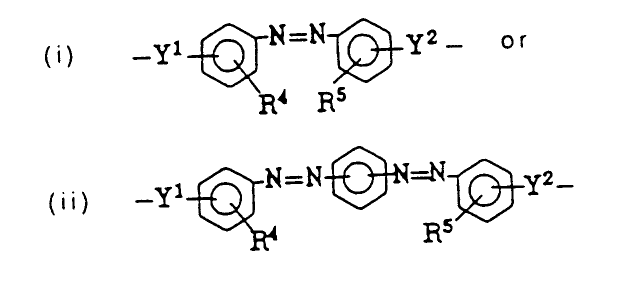 wherein Y¹ and Y², which may be the same or different, represent oxygen, imino (-NH-) or a group of the formula:
*R⁶-O, *R⁶-NH, O-R⁶-O, *NHCO-R⁶-NH or *CONH-R⁶-NH;
wherein R⁶ is alkylene and the atom or the end of the group marked by * is bonded to the phenyl ring;
R⁴ and R⁵ are each, independently, hydrogen, halogen, nitro or phenyl;
R² is a polyalkylene glycol residue;
Z is oxygen or imino and both groups Z are the same;
R³ is alkylene or a group of the formula:
R⁷-O-R⁸, R⁷-NH-R⁸ or 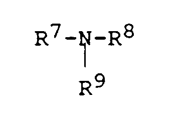 wherein R⁷ and R⁸, which may be the same or different, are alkylene; and
R⁹ is alkyl;
each segment of the said polyurethane being contained in the proportion of x, y and z and being a block-type polymer random type polymer, or a combination thereof, which comprises:
(i) mixing an azobenzene monomer having two functional groups, which gives the structural unit B (azo compound (B)) with at least one of a polymer which gives the structural unit C (polymer (C)) and a diol compound which gives the structural unit D (diol compound (D)) in appropriate molar proportions, and then subjecting the resulting mixture to a polyaddition reaction with a diisocyanate which gives the structural unit A (diisocyanate (A)) in amounts equimolar with the sum of three preceding compounds; or (ii) reacting azo compound (B) and equimolecular diisocyanate (A), and at least one of (a) reacting polymer (C) and equimolecular diisocyanate (A), and (b) reacting diol compound (D) and equimolecular diisocyanate (A), and optionally mincing the resulting mixture of block copolymers thus obtained.

2. A process according to claim 1 in which R¹ is a C₂₋₉ alkylene, an aromatic group of the formula: 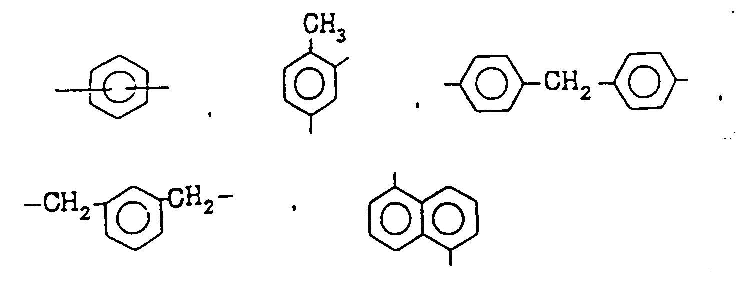 or an alicylic groups of the formula:

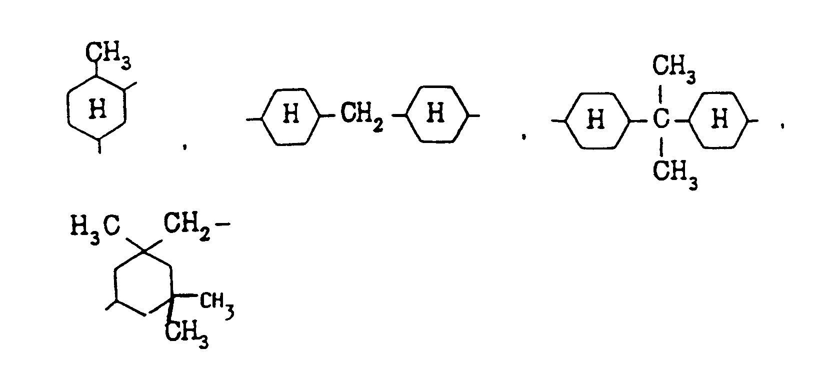

3. A process according to claim 1 or 2 in which the diisocyanate residue, unit A, is the residue of tetramethylene diisocyanate, hexamethylene diisocyanate, 2,2,5-trimethylhexamethylene diisocyanate, octamethylene diisocyanate, o-, m- or p-phenylene diisocyanate, 2,4- or 2,6-tolylene diisocyanate (TDI), 4,4'-diphenylmethane diisocyanate (MDI), o-, m- or p-xylylene diisocyanate, naphthalene 1,5-diisocyanate, hydrogenated TDI, hydrogenated MDI, dicyclohexyldimethylmethane 4,4'-diisocyanate, or isophorone diisocyanate.

4. A process according to claim 1 or 2, wherein R¹ is C4-8 alkylene, or a divalent group of a benzene or cyclohexyl, both which may be optionally substituted by one to four methyl groups, and R¹ may be linked to the cyanate residues either directly, or when present, optionally via a methyl group.

5. A process according to claim 4 wherein R¹ is hexamethylene, 3,5,5-trimethylcyclohexanly-1-yl-3 ylmethylene, or xylylene.

6. A process according to any one of the preceding claims wherein aza is a group of the formula: 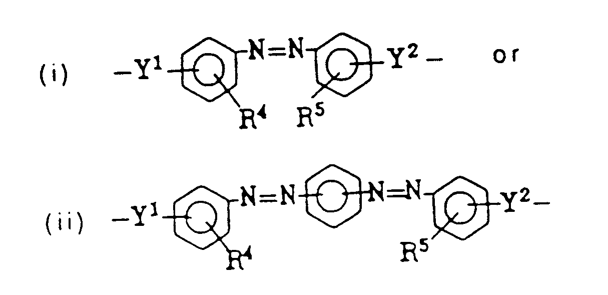 wherein Y¹ and Y², which may be the same or different, are oxygen, imino or a group of the formula: *R⁶-O or *R⁶-NH (in which R⁶ is C1-4 alkylene) and R⁴ and R⁵ are hydrogen.

7. A process according to any one of the preceding claims wherein R² is a polymer residue of C1-4 alkylene glycol.

8. A process according to any one of the preceding claims wherein R³ is C1-6 alkylene or a group of the formula: R⁷-O-R⁸ (in which R⁷ and R⁸, which may be the same or different, are C1-6 alkylene).

9. A process according to any one of the preceding claims wherein proportion of the segments, x : y is less than 0.6: more than 0.4, x : z is less than 0.2 : more than 0.8, or x : y : z is less than 0.2 : less than 0.1 : more than 0.7.

10. A process according to claim 1, wherein R¹ is a group of the formula: 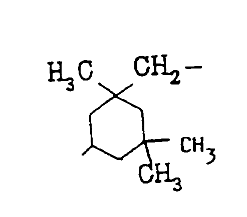 and R¹ in each of the segments A-B, A-C and A-D are the same,
aza is a group of the formula: 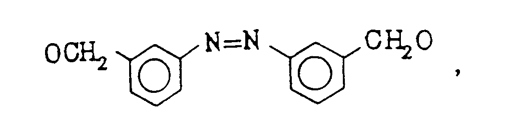 Z-R²-Z is a polyethylene glycol residue and R³ is 1,2-propylene.

## Patentansprüche (Patentansprüche für folgende(n) Vertragsstaat(en): AT, BE, CH, DE, DK, FR, GB, IT, LI, LU, NL, SE)

1. Polyurethan (I) eines durchschnittlichen Molekulargewichts von 1000 bis 100000, umfassend mehrere Abschnitte, bei denen es sich jeweils um Struktureinheiten A-B, A-C und A-D der Formeln: 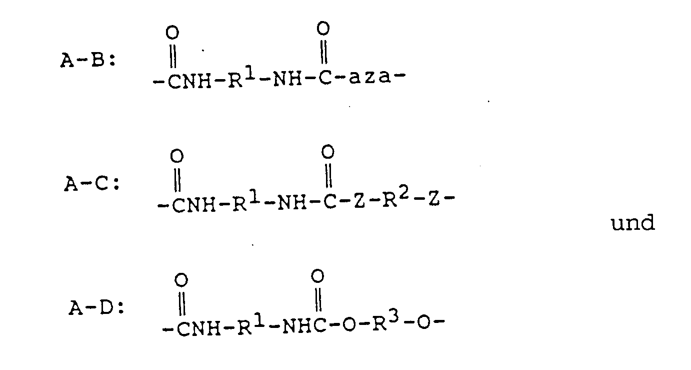 handelt, wobei das Molverhältnis der Segmente A-B, A-C und A-D, d.h. x/y/z, 0,01 bis 0,8/0 bis 0,80/0 bis 0,99 beträgt, wobei gilt, daß die Summe von x, y und z = 1,0; wobei es sich bei den Abschnitten A, B, C und D um Struktureinheiten der Formeln: 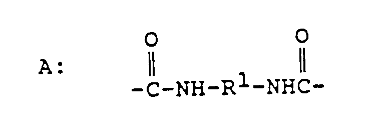
B: -aza- und
C: -Z-R²-Z-
D: -O-R³-O-;
handelt, worin bedeuten:
R¹ ein Skelett eines Diisocyanats, wobei die drei R¹-Gruppen in jedem der Abschnitte A-B, A-C und A-D gleich oder verschieden sein können;
aza eine Gruppe der Formel: 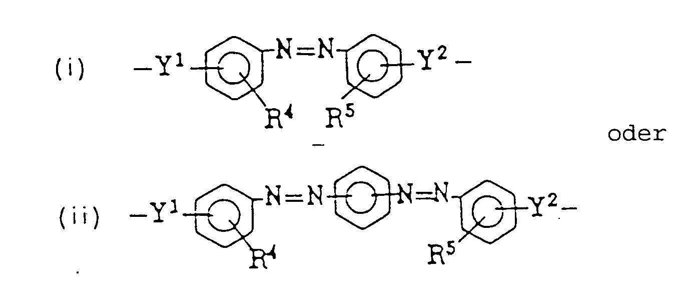 Y¹ und Y², die gleich oder verschieden sein können, Sauerstoff, Imino (-NH-) oder eine Gruppe der Formel:
*R⁶-O, *R⁶-NH, O-R⁶-O, *NHCO-R⁶-NH oder *CONH-R⁶-NH;
R⁶ Alkylen, wobei das mit * markierte Atom oder Ende der Gruppe an den Phenylring gebunden ist;
R⁴ und R⁵ jeweils unabhängig voneinander Wasserstoff, Halogen, Nitro oder Phenyl;
R² einen Polyalkylenglycolrest;
Z Sauerstoff oder Imino, wobei beide Gruppen Z dieselben sind;
R³ Alkylen oder eine Gruppe der Formel:
R⁷-O-R⁸, R⁷-NH-R⁸ oder 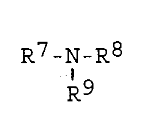 R⁷ und R⁸, die gleich oder verschieden sein können, jeweils Alkylen und
R⁹ Alkyl, und wobei jeder Abschnitt des Polyurethans im Mengenanteil x, y und z vorhanden ist und aus einem blockartigen Polymer, einem willkürlichen Polymer oder einer Kombination hiervon besteht.

2. Polyurethan nach Anspruch 1, wobei R¹ für ein C₂₋₉-Alkylen, eine aromatische Gruppe der Formel: 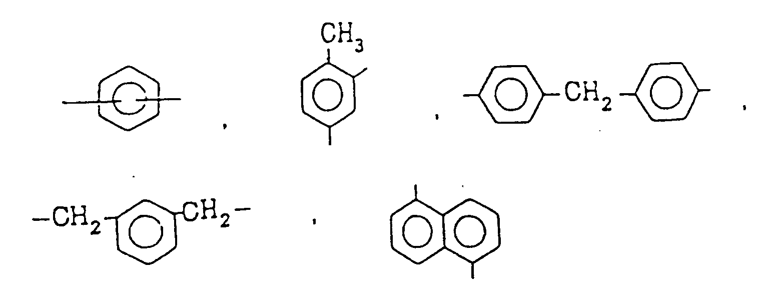 oder eine alicyclische Gruppe der Formel: 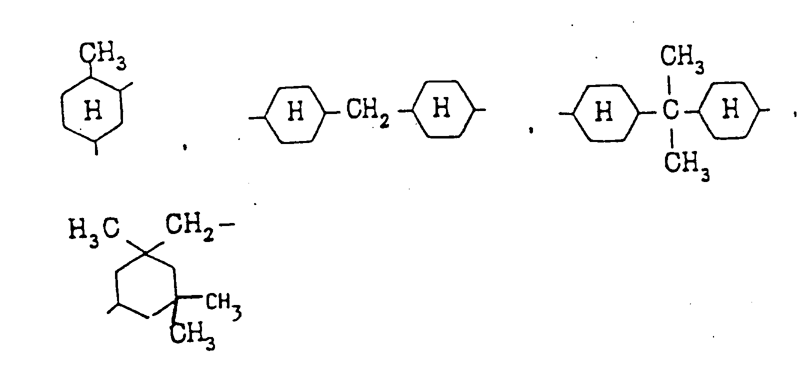 steht.

3. Polyurethanmasse nach Anspruch 1 oder 2, wobei der Diisocyanatrest, Einheit A, der Rest von Tetramethylendiisocyanat, Hexamethylendiisocyanat, 2,2,5-Trimethylhexamethylendiisocyanat, Octamethylendiisocyanat, o-, m- oder p-Phenylendiisocyanat, 2,4- oder 2,6-Tolylendiisocyanat (TDI), 4,4'-Diphenylmethandiisocyanat (MDI), o-, m- oder p-Xylylendiisocyanat, Naphthalin-1,5-diisocyanat, hydriertem TDI, hydriertem MDI, Dicyclohexyldimethylmethan-4,4'-diisocyanat oder Isophorondiisocyanat ist.

4. Polyurethan nach Anspruch 1 oder 2, wobei R¹ für C₄₋₈-Alkylen oder eine zweiwertige Gruppe eines Benzols oder Cyclohexans, die gegebenenfalls ein- bis vierfach methylsubstituiert sein können, steht und R¹ entweder direkt oder - falls vorhanden - gegebenenfalls über eine Methylgruppe an die Cyanatreste gebunden ist.

5. Polyurethan nach Anspruch 4, wobei R¹ für Hexamethylen, 3,5,5-Trimethylcyclohexan-1-yl-3-ylmethylen oder Xylylen steht.

6. Polyurethan nach einem der vorhergehenden Ansprüche, wobei aza für eine Gruppe der Formel: 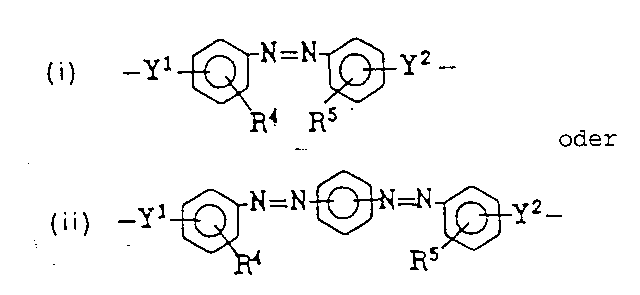 worin Y¹ und Y², die gleich oder verschieden sein können, Sauerstoff, Imino oder eine Gruppe der Formel: *R⁶-O oder *R⁶-NH (mit R⁶ gleich C₁₋₄-Alkylen) bedeuten und R⁴ und R⁵ Wasserstoff darstellen, steht.

7. Polyurethan nach einem der vorhergehenden Ansprüche, wobei R² für einen Polymerrest von C₁₋₄-Alkylenglycol steht.

8. Polyurethan nach einem der vorhergehenden Ansprüche, wobei R³ für C₁₋₆-Alkylen oder eine Gruppe der Formel: R⁷-O-R⁸ (mit R⁷ und R⁸, die gleich oder verschieden sein können, gleich C₁₋₆-Alkylen) steht.

9. Polyurethan nach einem der vorhergehenden Ansprüche, wobei das Verhältnis der Abschnitte x/y kleiner 0,6/ größer 0,4, x/z kleiner 0,2/ größer 0,8 oder x/y/z kleiner 0,2/ kleiner 0,1/ größer 0,7 ist.

10. Polyurethan nach Anspruch 1, wobei R¹ für eine Gruppe der Formel: 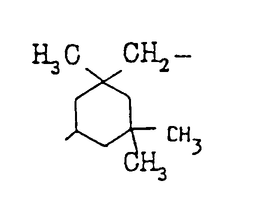 steht und R¹ in jedem der Segmente A-B, A-C und A-D die gleiche Bedeutung besitzt;
aza eine Gruppe der Formel: 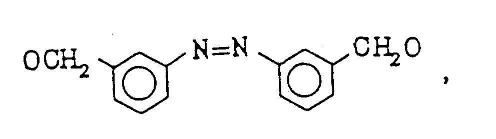 darstellt, Z-R²-Z einen Polyethylenglycolrest bedeutet und R³ 1,2-Propylen entspricht.

11. Verfahren zur Herstellung des Polyurethans (I) gemäß Anspruch 1 durch (i) Vermischen eines die Struktureinheit B (Azoverbindung (B)) liefernden Azobenzolmonomeren mit zwei funktionellen Gruppen mit mindestens einem die Struktureinheit C (Polymer (C)) liefernden Polymer und einer die Struktureinheit D (Diolverbindung (D)) liefernden Diolverbindung in geeigneten molaren Mengen und anschließende Polyaddition des erhaltenen Gemischs mit einem die Struktureinheit A (Diisocyanat (A)) liefernden Diisocyanat in äquimolaren Mengen zur Summe der drei zuvor genannten Verbindungen oder (ii) Umsetzen von Azoverbindung (B) und äquimolarem Diisocyanat (A) und mindestens eine der Maßnahmen (a) Umsetzen von Polymer (C) und äquimolarem Diisocyanat (A) und (b) Umsetzen von Diolverbindung (D) und äquimolarem Diisocyanat (A), und gegebenenfalls Zerkleinern des erhaltenen Gemischs der dabei gebildeten Blockcopolymeren.

12. Pharmazeutisches Hilfsmittel mit einem Polyurethan nach einem der Ansprüche 1 bis 10.

## Patentansprüche (Patentansprüche für folgende(n) Vertragsstaat(en): ES, GR)

1. Verfahren zur Herstellung von Polyurethanen (I) eines durchschnittlichen Molekulargewichts von 1000 bis 100000, umfassend mehrere Abschnitte, bei denen es sich jeweils um Struktureinheiten A-B, A-C und A-D der Formeln: 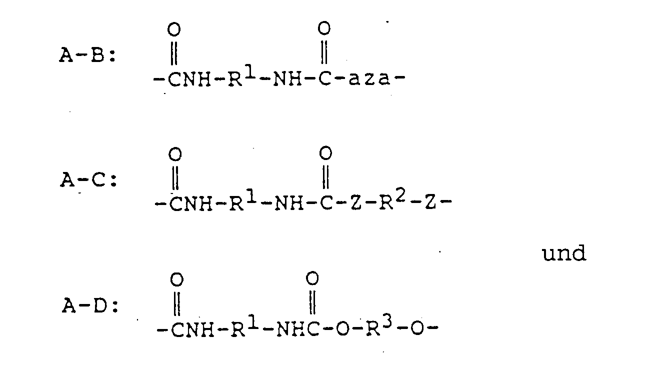 handelt, wobei das Molverhältnis der Segmente A-B, A-C und A-D, d.h. x/y/z, 0,01 bis 0,8/0 bis 0,80/0 bis 0,99 beträgt, wobei gilt, daß die Summe von x, y und z = 1,0; wobei es sich bei den Abschnitten A, B, C und D um Struktureinheiten der Formeln: 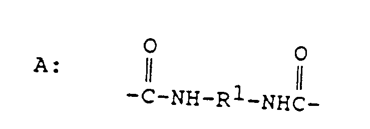
B: -aza-
C: -Z-R²-Z- und
D: -O-R³-O-;
handelt, worin bedeuten:
R¹ ein Skelett eines Diisocyanats, wobei die drei R¹-Gruppen in jedem der Abschnitte A-B, A-C und A-D gleich oder verschieden sein können; aza eine Gruppe der Formel: 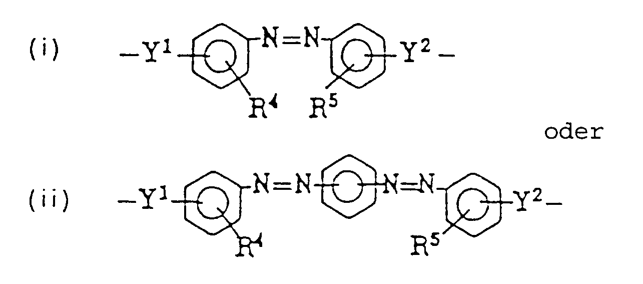
Y¹ und Y², die gleich oder verschieden sein können, Sauerstoff, Imino (-NH-) oder eine Gruppe der Formel:
*R⁶-O, *R⁶-NH, O-R⁶-O, *NHCO-R⁶-NH oder *CONH-R⁶-NH;
R⁶ Alkylen, wobei das mit * markierte Atom oder Ende der Gruppe an den Phenylring gebunden ist;
R⁴ und R⁵ jeweils unabhängig voneinander Wasserstoff, Halogen, Nitro oder Phenyl;
R² einen Polyalkylenglycolrest;
Z Sauerstoff oder Imino, wobei beide Gruppen Z dieselben sind;
R³ Alkylen oder eine Gruppe der Formel:
R⁷-O-R⁸, R⁷-NH-R⁸ oder 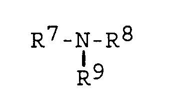 R⁷ und R⁸, die gleich oder verschieden sein können, jeweils Alkylen und
R⁹ Alkyl, und wobei jeder Abschnitt des Polyurethans im Mengenanteil x, y und z vorhanden ist und aus einem blockartigen Polymer, einem willkürlichen Polymer oder einer Kombination hiervon besteht, durch
(i) Vermischen eines die Struktureinheit B (Azoverbindung (B)) liefernden Azobenzolmonomeren mit zwei funktionellen Gruppen mit mindestens einem die Struktureinheit C (Polymer (C)) liefernden Polymer und einer die Struktureinheit D (Diolverbindung (D)) liefernden Diolverbindung in geeigneten molaren Mengen und anschließende Polyaddition des erhaltenen Gemischs mit einem die Struktureinheit A (Diisocyanat (A)) liefernden Diisocyanat in äquimolaren Mengen zur Summe der drei zuvor genannten Verbindungen oder (ii) Umsetzen von Azoverbindung (B) und äquimolarem Diisocyanat (A) und mindestens eine der Maßnahmen (a) Umsetzen von Polymer (C) und äquimolarem Diisocyanat (A) und (b) Umsetzen von Diolverbindung (D) und äquimolarem Diisocyanat (A), und gegebenenfalls Zerkleinern des erhaltenen Gemischs der dabei gebildeten Blockcopolymeren.

2. Verfahren nach Anspruch 1, worin R¹ für ein C₂₋₉-Alkylen, eine aromatische Gruppe der Formel: 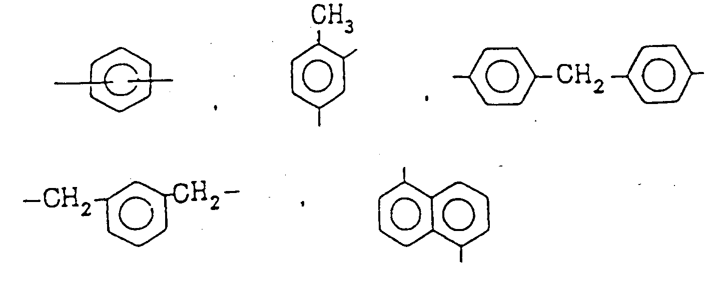 oder eine alicyclische Gruppe der Formel: 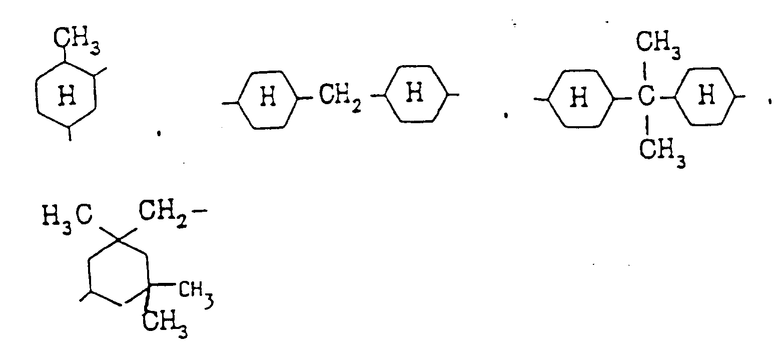 steht.

3. Verfahren nach Anspruch 1 oder 2, worin der Diisocyanatrest, Einheit A, der Rest von Tetramethylendiisocyanat, Hexamethylendiisocyanat, 2,2,5-Trimethylhexamethylendiisocyanat, Octamethylendiisocyanat, o-, m- oder p-Phenylendiisocyanat, 2,4- oder 2,6-Tolylendiisocyanat (TDI), 4,4'-Diphenylmethandiisocyanat (MDI), o-, m- oder p-Xylylendiisocyanat, Naphthalin-1,5-diisocyanat, hydriertem TDI, hydriertem MDI, Dicyclohexyldimethylmethan-4,4'-diisocyanat oder Isophorondiisocyanat ist.

4. Verfahren nach Anspruch 1 oder 2, worin R¹ für C₄₋₈-Alkylen oder eine zweiwertige Gruppe eines Benzols oder Cyclohexans, die gegebenenfalls ein- bis vierfach methylsubstituiert sein können, steht und R¹ entweder direkt oder - falls vorhanden - gegebenenfalls über eine Methylgruppe an die Cyanatreste gebunden ist.

5. Verfahren nach Anspruch 4, worin R¹ für Hexamethylen, 3,5,5-Trimethylcyclohexan-1-yl-3-ylmethylen oder Xylylen steht.

6. Verfahren nach einem der vorhergehenden Ansprüche, worin aza für eine Gruppe der Formel: 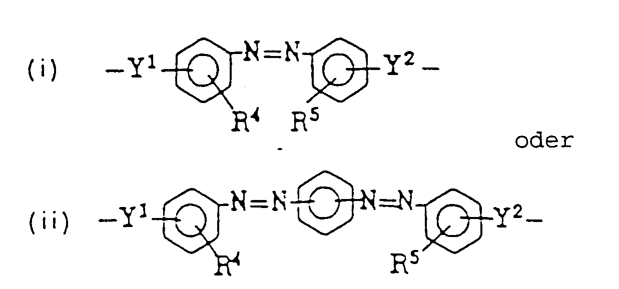 worin Y¹ und Y², die gleich oder verschieden sein können, Sauerstoff, Imino oder eine Gruppe der Formel: *R⁶-O oder *R⁶-NH (mit R⁶ gleich C₁₋₄-Alkylen) bedeuten und R⁴ und R⁵ Wasserstoff darstellen, steht.

7. Verfahren nach einem der vorhergehenden Ansprüche, worin R² für einen Polymerrest von C₁₋₄-Alkylenglycol steht.

8. Verfahren nach einem der vorhergehenden Ansprüche, worin R³ für C₁₋₆-Alkylen oder eine Gruppe der Formel: R⁷-O-R⁸ (mit R⁷ und R⁸, die gleich oder verschieden sein können, gleich C₁₋₆-Alkylen) steht.

9. Verfahren nach einem der vorhergehenden Ansprüche, worin das Verhältnis der Abschnitte x/y kleiner 0,6/ größer 0,4, x/z kleiner 0,2/ größer 0,8 oder x/y/z kleiner 0,2/ kleiner 0,1/ größer 0,7 ist.

10. Verfahren nach Anspruch 1, worin R¹ für eine Gruppe der Formel: 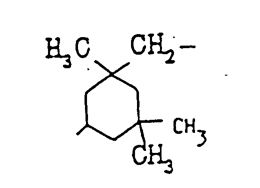 steht und R¹ in jedem der Segmente A-B, A-C und A-D die gleiche Bedeutung besitzt;
aza eine Gruppe der Formel: 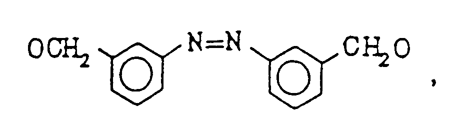 darstellt, Z-R²-Z einen Polyethylenglycolrest bedeutet und R³ 1,2-Propylen entspricht.

## Revendications (Revendications pour l'(les) Etat(s) contractant(s) suivant(s): AT, BE, CH, DE, DK, FR, GB, IT, LI, LU, NL, SE)

1. Polyuréthannes (I) de masse moléculaire moyenne de 1000 à 100000, comprenant plusieurs segments, qui sont chacun des motifs structurels A-B, A-C et A-D de formules: 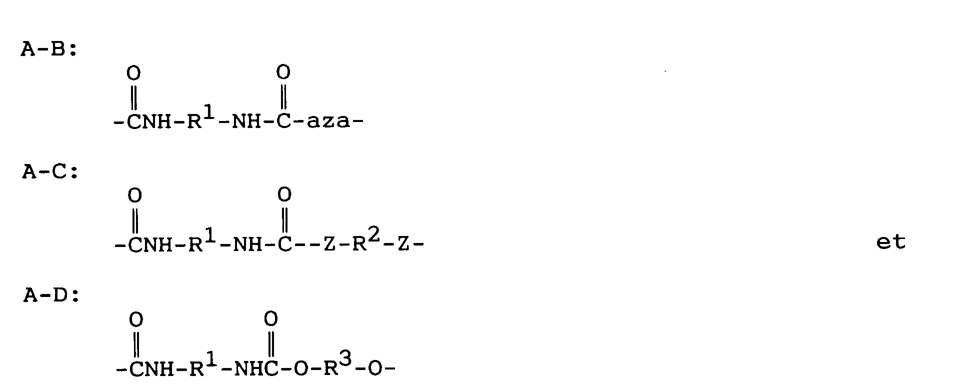 la proportion molaire desdits segments de A-B, A-C et A-D, à savoir x:y:z étant de 0,01 à 0,8:0 à 0,80:0 à 0,99, à condition que la somme de x, y et z vaille 1,0;
les segments A, B, C et D étant des motifs structuraux de formules: 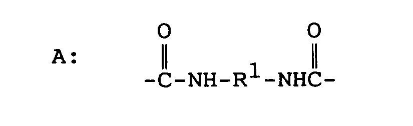
B: -aza-
C: -Z-R²-Z- et
D: -O-R³-O-;
dans lesquelles R¹ est un squelette de diisocyanate et les trois groupes R¹ dans chacun des segments A-B, A-C et A-D sont identiques ou différents;
aza est un groupe de formule: 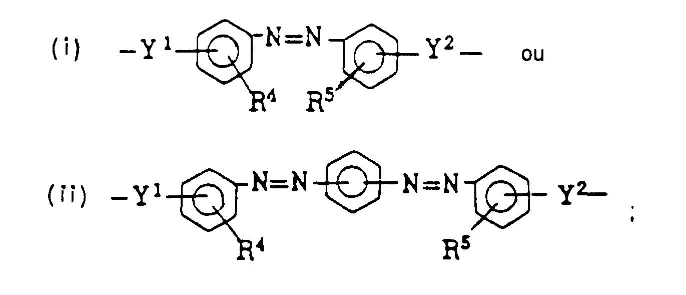 dans laquelle Y¹ et Y², qui peuvent être identiques ou différents, représentent un radical oxygène, imino (-NH-) ou un groupe de formules:
*R⁶-O, *R⁶-NH, O-R⁶-O, *NHCO-R⁶-NH ou *CONH-R⁶-NH;
dans lesquelles R⁶ est un groupe alkylène et l'atome ou la terminaison du groupe marqué par * est lié au noyau phényle;
R⁴ et R⁵ sont chacun, indépendamment, un radical hydrogène, halogène, nitro ou phényle;
R² est un résidu polyalkylèneglycol;
Z est un radical oxygène ou imino et les deux groupes Z sont identiques;
R³ est un groupe alkylène ou un groupe de formule:
R⁷-O-R⁸, R⁷-NH-R⁸ ou 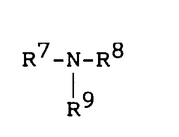 dans lesquelles R⁷ et R⁸, qui peuvent identiques ou différents, sont un groupe alkylène; et
R⁹ est un groupe alkyle,
chaque segment dudit polyuréthanne étant contenu dans la proportion de x, y et z, et étant un polymère de type séquencé, un polymère de type statistique ou une combinaison de ceux-ci.

2. Polyuréthanne selon la revendication 1, dans lequel R¹ est un groupe alkylène en C₂ à C₉, un groupe aromatique de formule: 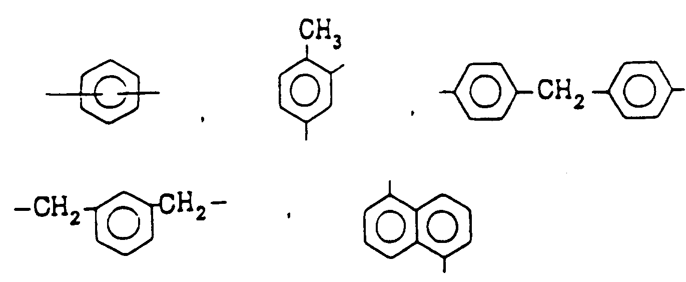 ou un groupe alicyclique de formule:

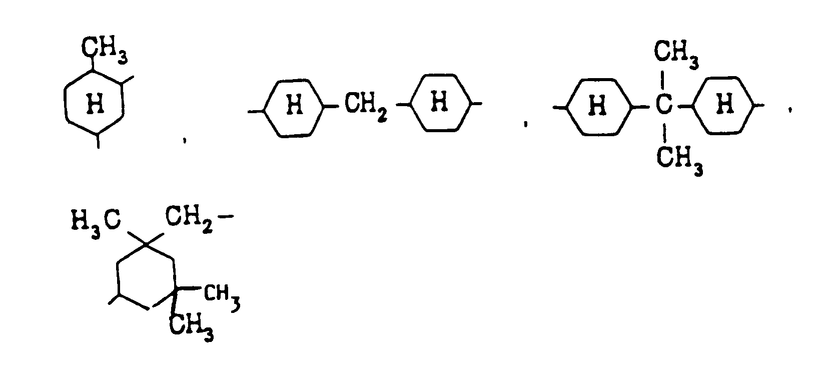

3. Composition de polyuréthanne selon la revendication 1 ou 2, dans laquelle le résidu diisocyanate, le motif A, est le résidu du diisocyanate de tétraméthylène, du diisocyanate d'hexaméthylène, du diisocyanate de 2,2,5-triméthylhexaméthylène, du diisocyanate d'octaméthylène, de diisocyanate d'o-, m- ou p-phénylène, du diisocyanate de 2,4- ou 2,6-tolylène (TDI), du diisocyanate de 4,4'-diphénylméthane (MDI), du diisocyanate de o-, m- ou p-xylylène, du naphtalène 1,5-diisocyanate, du TDI hydrogéné, du MDI hydrogéné, du dicyclohexyldiméthylméthane 4,4'-diisocyanate et du diisocyanate d'isophorone.

4. Polyuréthanne selon la revendication 1 ou 2, dans lequel R¹ est un groupe alkylène en C₄ à C₈, ou un groupe divalent d'un benzène ou cyclohexyle, dont les deux peuvent éventuellement substitués par un à quatre groupes méthyle, et R¹ peut être lié aux résidus cyanate soit directement, ou quand il est présent, éventuellement par l'intermédiaire d'un groupe méthyle.

5. Polyuréthanne selon la revendication 4, dans lequel R¹ est l'hexaméthylène, le 3,5,5-triméthylcyclohexane-1-yl-3-ylméthylène ou le xylylène.

6. Polyuréthanne selon l'une quelconque des revendications précédentes, dans lequel le groupe aza est un groupe de formule 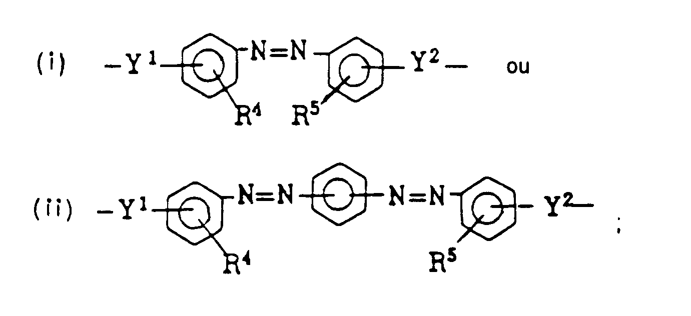 dans laquelle Y¹ et Y², qui peuvent être identiques ou différents, sont un résidu oxygène, imino, ou un groupe de formule *R⁶-o ou *R⁶-NH (dans laquelle R⁶ est un alkylène en C₁ à C₄) et R⁴ et R⁵ sont des atomes d'hydrogène.

7. Polyuréthanne selon l'une quelconque des revendications précédentes, dans lequel R² est un résidu polymère d'alkylèneglycol en C₁ à C₄.

8. Polyuréthanne selon l'une quelconque des revendications précédentes, dans lequel R³ est un groupe alkylène en C₁ à C₆ ou un groupe de formule R⁷-O-R⁸ (dans laquelle R⁷ et R⁸, qui peuvent être identiques ou différents, sont un groupe alkylène en C₁ à C₆).

9. Polyuréthanne selon l'une quelconque des revendications précédentes, dans lequel la proportion des segments x:y est moins de 0,6:plus de 0,4, x:z est moins de 0,2:plus de 0,8, ou x:y:z est moins de 0,2:moins de 0,1:plus de 0,7.

10. Polyuréthanne selon la revendication 1, dans lequel R¹ est un groupe de formule: 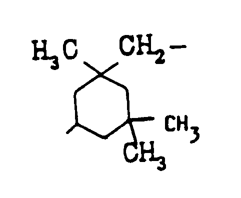 et R¹ dans chacun des segments A-B, A-C et A-D sont identiques,
aza est un groupe de formule: 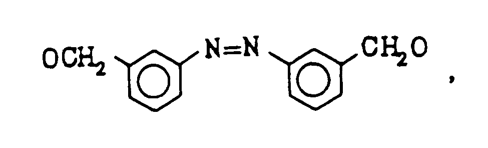 Z-R²-Z est un résidu polyéthylèneglycol et R³ est un groupe 1,2-propylène.

11. Procédé pour la préparation du polyuréthanne (I) selon la revendication 1, qui consiste:
(i) à mélanger un monomère azobenzène ayant deux groupes fonctionnels, qui donnent le motif structurel B (composé azo (B)) avec au moins un d'un polymère qui donne le motif structurel C (polymère(C)) et d'un composé diol qui donne le motif structurel D (composé diol (D)) dans des proportions molaires appropriées, puis à soumettre le mélange résultant à une réaction de polyaddition avec un diisocyanate qui donne le motif structurel A (diisocyanate (A)) en quantités équimolaires avec la somme des trois composés précédents;
ou
(ii) à faire réagir le composé azo (B) et un diisocyanate (A) en une quantité équimoléculaire, et au moins une opération consistant (a) à faire réagir le polymère (C) et le diisocyanate (A) en une quantité équimoléculaire, et (b) à faire réagir le composé diol (D) et le diisocyanate (A) en une quantité équimoléculaire, et éventuellement à hacher le mélange résultant de copolymères séquencés ainsi obtenus.

12. Excipient pharmaceutique contenant un polyuréthanne selon l'une quelconque des revendications 1 à 10.

## Revendications (Revendications pour l'(les) Etat(s) contractant(s) suivant(s): ES, GR)

1. Procédé pour la préparation de polyuréthannes (I) de masse moléculaire de 1000 à 100000, comprenant plusieurs segments, qui sont chacun des motifs structurels A-B, A-C et A-D de formules: 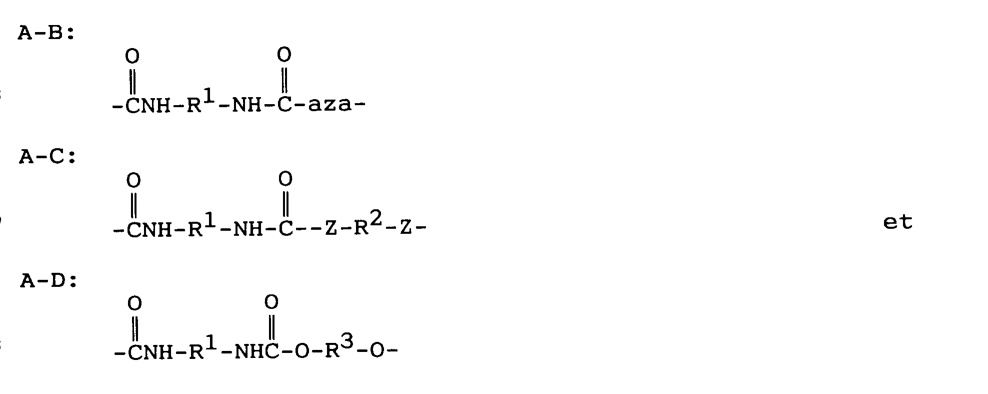 la proportion molaire desdits segments de A-B, A-C et A-D, à savoir x:y:z étant de 0,01 à 0,8:0 à 0,80:0 à 0,99, à condition que la somme de x, y et z vaille 1,0;
les segments A, B, C et D étant des motifs structuraux de formules: 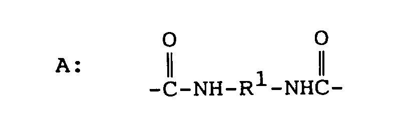
B: -aza-
C: -Z-R²-Z- et
D: -O-R³-O-;
dans lesquelles R¹ est un squelette de diisocyanate et les trois groupes R¹ dans chacun des segments A-B, A-C et A-D sont identiques ou différents;
aza est un groupe de formule: 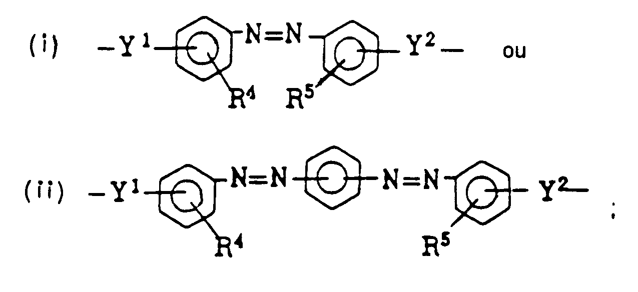 dans laquelle Y¹ et Y², qui peuvent être identiques ou différents, représentent un radical oxygène, imino (-NH-) ou un groupe de formules:
*R⁶-O, *R⁶-NH, O-R⁶-O, *NHCO-R⁶-NH ou *CONH-R⁶-NH;
dans lesquelles R⁶ est un groupe alkylène et l'atome ou la terminaison du groupe marqué par * est lié au noyau phényle;
R⁴ et R⁵ sont chacun, indépendamment, un radical hydrogène, halogène, nitro ou phényle;
R² est un résidu polyalkyléneglycol;
Z est un radical oxygène ou imino et les deux groupes Z sont identiques;
R³ est un groupe alkylène ou un groupe de formule:
R⁷-O-R⁸, R⁷-NH-R⁸ ou 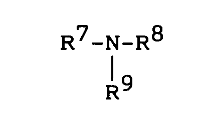 dans lesquelles R⁷ et R⁸, qui peuvent identiques ou différents, sont un groupe alkylène; et
R⁹ est un groupe alkyle,
chaque segment dudit polyuréthanne étant contenu dans la proportion de x, y et z, et étant un polymère de type séquencé, un polymère de type statistique ou une combinaison de ceux-ci qui consiste:
(i) à mélanger un monomère azobenzène ayant deux groupes fonctionnels, qui donnent le motif structurel B (composé azo (B)) avec au moins un d'un polymère qui donne le motif structurel C (polymère(C)) et d'un composé diol qui donne le motif structurel D (composé diol (D)) dans des proportions molaires appropriées, puis à soumettre le mélange résultant à une réaction de polyaddition avec un diisocyanate qui donne le motif structurel A (diisocyanate (A)) en quantités équimolaires avec la somme des trois composés précédents;
ou
(ii) à faire réagir le composé azo (B) et un diisocyanate (A) en une quantité équimoléculaire, et au moins une opération consistant (a) à faire réagir le polymère (C) et le diisocyanate (A) en une quantité équimoléculaire, et (b) à faire réagir le composé diol (D) et le diisocyanate (A) en une quantité équimoléculaire, et éventuellement à hacher le mélange résultant de copolymères séquencés ainsi obtenus.

2. Procédé selon la revendication 1, dans lequel R¹ est un groupe alkylène en C₂ à C₉, un groupe aromatique de formule: 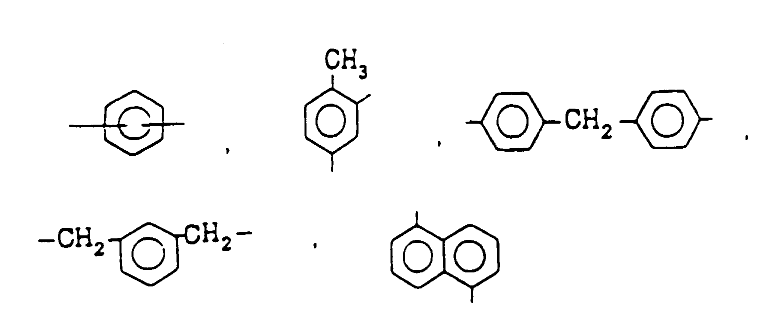 ou un groupe alicyclique de formule:

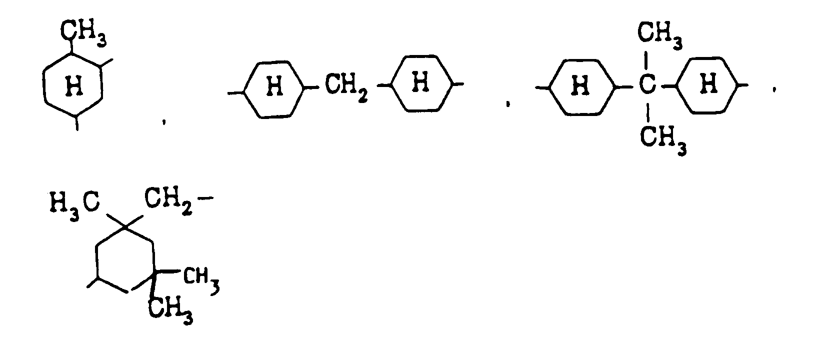

3. Procédé selon la revendication 1 ou 2, dans lequel le résidu diisocyanate, le motif A, est le résidu du diisocyanate de tétraméthylène, du diisocyanate d'hexaméthylène, du diisocyanate de 2,2,5-triméthylhexaméthylène, du diisocyanate d'octaméthylène, du diisocyanate d'o-, m- ou p-phénylène, du diisocyanate de 2,4- ou 2,6-tolylène (TDI), du diisocyanate de 4,4'-diphénylméthane (MDI), du diisocyanate de o-, m- ou p-xylylène, du naphtalène 1,5-diisocyanate, du TDI hydrogéné, du MDI hydrogéné, du dicyclohexyldiméthylméthane 4,4'-diisocyanate et du diisocyanate d'isophorone.

4. Procédé selon la revendication 1 ou 2, dans lequel R¹ est un groupe alkylène en C₄ à C₈, ou un groupe divalent d'un benzène ou cyclohexyle, dont les deux peuvent éventuellement substitués par un à quatre groupes méthyle, et R¹ peut être lié aux résidus cyanate soit directement, soit quand il est présent, éventuellement par l'intermédiaire d'un groupe méthyle.

5. Procédé selon la revendication 4, dans lequel R¹ est l'hexaméthylène, le 3,5,5-triméthylcyclohexane-1-yl-3-ylméthylène ou le xylylène.

6. Procédé selon l'une quelconque des revendications précédentes, dans lequel le groupe aza est un groupe de formule 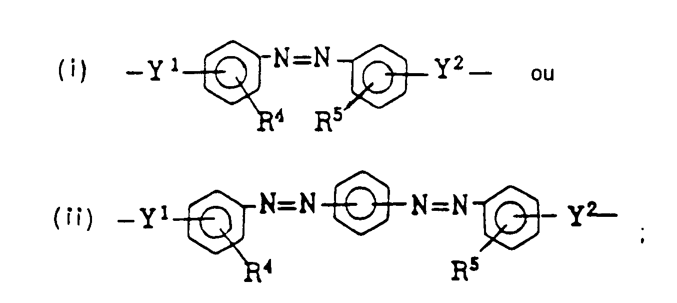 dans laquelle Y¹ et Y², qui peuvent être identiques ou différents, sont un résidu oxygène, imino, ou un groupe de formule *R⁶-o ou *R⁶-NH (dans laquelle R⁶ est un alkylène en C₁ à C₄) et R⁴ et R⁵ sont des atomes d'hydrogène.

7. Procédé selon l'une quelconque des revendications précédentes, dans lequel R² est un résidu polymère d'alkylèneglycol en C₁ à C₄.

8. Procédé selon l'une quelconque des revendications précédentes, dans lequel R³ est un groupe alkylène en C₁ à C₆ ou un groupe de formule R⁷-O-R⁸ (dans laquelle R⁷ et R⁸, qui peuvent être identiques ou différents, sont un groupe alkylène en C₁ à C₆).

9. Procédé selon l'une quelconque des revendications précédentes, dans lequel la proportion des segments x:y est moins de 0,6:plus de 0,4; x:z est moins de 0,2:plus de 0,8, ou x:y:z est moins de 0,2:moins de 0,1:plus de 0,7.

10. Polyuréthanne selon la revendication 1, dans lequel R¹ est un groupe de formule: 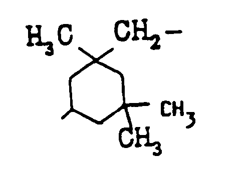 et R¹ dans chacun des segments A-B, A-C et A-D sont identiques,
aza est un groupe de formule: 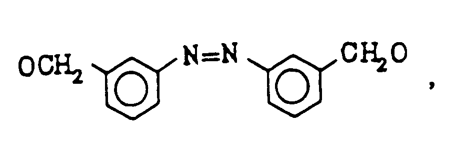 Z-R²-Z est un résidu polyéthylèneglycol et R³ est un groupe 1,2-propylène.
